# EUROPEAN PATENT SPECIFICATION

(11) **EP 0 946 727 B1**
(45) Date of publication and mention of the grant of the patent: **24.01.2007**
(21) Application number: 97947371.7
(22) Date of filing: 29.10.1997
(51) Int. Cl.: C12N 15/13, C07K 16/00, C07K 16/28, C07K 16/46, C12N 1/20

(54) **ANTIBODY MUTANTS**
ANTIKÖRPERMUTANTE
MUTANTS D'ANTICORPS

(30) Priority: 27.11.1996 US 756150
(43) Date of publication of application: 06.10.1999
(73) Proprietor: GENENTECH, INC., South San Francisco, CA 94080-4990 (US)
(72) Inventor: JARDIEU, Paula, M., San Francisco, CA 94109 (US); PRESTA, Leonard, G., San Francisco, CA 94109 (US)
(74) Representative: Kremer, Simon Mark
(86) International application number: PCT/US1997/020169
(87) International publication number: WO 1998/023746

(56) References cited:
- EP-A- 0 699 755
- WO-A-96/36359
- J. HILDRETH ET AL.: "A human lymphocyte-associated antigen involved in cell-mediated lympholysis." EUROPEAN JOURNAL OF IMMUNOLOGY, vol. 13, no. 3, March 1983, WEINHEIM, GERMANY, pages 202-208, XP002054055 cited in the application
- R. SABAN ET AL.: "Humanized anti-IgE monoclonal antibody Mae25 blocks passive sensitization of rhesus monkey bladder in vitro." THE FASEB JOURNAL, vol. 8, no. 5, 18 March 1994, BETHESDA, MD, USA, page A682 XP002063118
- W. WERTHER ET AL.: "Humanization of an anti-lymphocyte function-associated antigen (LFA)-1 monoclonal antibody and reengineering of the humanized antibody for binding to rhesus LFA-1." THE JOURNAL OF IMMUNOLOGY, vol. 157, no. 11, 1 December 1996, BALTIMORE, MD, USA, pages 4986-4995, XP002054057

## Description

### BACKGROUND OF THE INVENTION

### Field of the Invention

This invention relates generally to the production of antibody mutants with beneficial properties from a therapeutic perspective.

### Description of Related Art

It is now over 100 years since animal antisera were first used therapeutically. Many of the drawbacks of the first attempts at human therapy using animal antisera were overcome by the development of monoclonal antibodies in the mid 1970's. Monoclonal antibodies are now commonly manufactured using recombinant DNA technology. Widespread use has been made of monoclonal antibodies, particularly those derived from rodents, however nonhuman antibodies are frequently antigenic in humans. The art has attempted to overcome this problem by constructing "chimeric" antibodies in which a nonhuman antigen-binding domain is coupled to a human constant domain (Cabilly *et al.,* U.S. patent No. 4,816,567). The isotype of the human constant domain may be selected to tailor the chimeric antibody for participation in antibody-dependent cellular cytotoxicity (ADCC) and complement-dependent cytotoxicity. In a further effort to resolve the antigen binding functions of antibodies and to minimize the use of heterologous sequences in human antibodies, Winter and colleagues (Jones *et al., Nature* 321:522-525 (1986); Riechmann *et al., Nature* 332:323-327 (1988); Verhoeyen *et al., Science* 239:1534-1536 (1988)) have substituted rodent complementarity determining region (CDR) residues for the corresponding segments of a human antibody to generate humanized antibodies. As used herein, the term "humanized" antibody is an embodiment of chimeric antibodies wherein substantially less than an intact human variable domain has been substituted by the corresponding sequence from a non-human species. In practice, humanized antibodies are typically human antibodies in which some CDR residues and possibly some framework region (FR) residues are substituted by residues from analogous sites in rodent antibodies.

Prior to administering the antibody to a human, preclinical work in nonhuman mammals is generally required or desired to evaluate the efficacy and/or toxicity of the antibody. For such studies, nonhuman primates are often the mammal of choice because they are the nearest relatives of humans and have many biologic, physiologic, biochemical and immunologic similarities to the human species. Furthermore, nonhuman primates develop many spontaneous diseases that are the counterpart to naturally occurring human diseases, and are susceptible to experimental infection with many bacterial, viral, parasitic and mitotic agents that cause disease in humans. In many instances, nonhuman primates represent the only nonhuman species in which these diseases occur spontaneously or can be induced experimentally. Monkeys of the macaque species, including the rhesus monkey and cynomolgus monkey, are particularly useful for preclinical studies. These animals have been frequently utilized for studies of infectious disease, immunity, hematopoiesis and transplantation.

### SUMMARY OF THE INVENTION

A problem associated with certain antibodies intended for therapeutic use is disclosed herein. In particular, the antibody for therapeutic use is normally generated using a target human antigen as the immunogen. The resultant antibody may be "species-dependent", *i.e.* while binding specifically to human antigen, it may be much less effective at binding a homologue of that antigen from a nonhuman mammal. This is found herein to be problematic, particularly where the nonhuman mammal is one in which preclinical studies of the antibody are to be carried out. An example is shown below for an anti-CD11a antibody used for treating LFA-1 mediated disorders. While the antibody had a strong binding affinity for human CD11a (*i*.*e*. 2 x 10⁻¹⁰ M), the affinity for the CD11a antigen from rhesus or cynomolgus monkey was unsuitable for generating preclinical data. Accordingly, a method for modifying a species-dependent antibody so as to confer the ability to bind to the antigen from the nonhuman mammal to a degree suitable for using the antibody mutant in a primate preclinical model has been developed herein. It is believed that this method can be applied to antibodies directed against other antigens. For example, in a study of 89 human leukocyte-specific monoclonal antibodies it was found that only 48 cross-reacted with rhesus determinants (Reimann *et al*. *Cytometry* 17:102-108 (1994)). In another study, it was found that three monoclonal antibodies against human erythropoietin had varying effectiveness against baboon erythropoietin (Wognum *et al*. *Exp*. *Hematol*. 18:228 (1990)). These antibodies displaying species-dependence serve as candidates for modification using the methods described herein. Species-dependence of antibodies will rely on differences in the protein sequences of the first and second mammalian species of interest and therefore may occur regardless of the source of the antibody, including human monoclonal antibodies derived from SCID mice or phage libraries (see discussion below concerning generation of such antibodies).

In one embodiment, the invention provides a method for making an antibody mutant, comprising the steps of:
(a) identifying hypervariable region residues in a species-dependent antibody which are involved in binding an antigen from a first mammalian species (e.g. a human) and those hypervariable region residues involved in binding a homologue of the antigen from a second different mammalian species (e.g. a nonhuman mammal);
(b) preparing a mutant of the species-dependent antibody wherein a residue identified in (a) as being involved in binding the antigen from the first mammalian species or the homologue thereof, or both, is replaced by another amino acid residue; and
(c) selecting an antibody mutant prepared in (b) which has a stronger binding affinity for the antigen from the second mammalian species than the species-dependent antibody. Step (b) can, for example, involve preparing a mutant of the species-dependent antibody wherein (I) a residue identified as being involved in binding the homologue, but not the antigen from the first mammalian species, is replaced by another amino add residue; (2) a residue identified as being involved in binding both the antigen from the first mammalian species and the homologue thereof is replaced by another amino acid residue; and/or (3) a residue identified. as being involved in binding the antigen from the first mammalian species, but not the homologue thereof, is replaced by another amino acid residue.

The invention further provides an antibody mutant of a species-dependent antibody which is MHMZ4 anti-human CD11a antibody which antibody mutant comprises an amino acid substitution in a hypervariable region of the species-dependent antibody and has a binding affinity for an antigen from a nonhuman mammal which is at least about 10 fold stronger than the binding affinity of the species-dependent antibody for the antigen. The antibody mutant binds CD11a from a nonhuman mammal such as rhesus CD11a and has a heavy chain variable domain comprising the amino acid sequence in SEQ ID NO:17, optionally combined with the humanized light chain variable domain in SEQ ID NO:2.

Various forms of the antibody mutant are contemplated herein. For example, the antibody mutant may be a full length antibody (*e.g.* having a human immunoglobulin constant region) or an antibody fragment (*e.g.* a F(ab')₂). Furthermore, the antibody mutant may be labeled with a detectable label, immobilized on a solid phase and/or conjugated with a heterologous compound (such as a cytotoxic agent).

Diagnostic and therapeutic uses for the antibody mutant are contemplated In one diagnostic application the antibody mutant may be used in a method for determining the presence of a protein of interest comprising exposing a sample suspected of containing the protein to the antibody mutant and determining binding of the antibody mutant to the sample. For this use, the antibody may be provided a kit comprising the antibody mutant and instructions for using the antibody mutant to detect the protein.

The invention further provides: isolated nucleic acid encoding the antibody mutant; a vector comprising the nucleic acid, optionally, operably linked to control sequences recognized by a host cell transformed with the vector; a host cell transformed with the vector; a process for producing the antibody mutant comprising culturing this host cell so that the nucleic acid is expressed and optionally, recovering the antibody mutant from the host cell culture (e.g. from the host cell culture medium).

The antibody may be provided as a composition comprising the antibody mutant and a pharmaceutically acceptable carrier or diluent. This composition for therapeutic use is sterile and may be lyophilized.

The antibody may be used in a method for treating a mammal comprising administering an effective amount of the antibody mutant to the mammal. The mammal to be treated in the method may be a nonhuman mammal, e.g. a primate suitable for gathering preclinical data. The nonhuman mammal may be healthy (e.g. in toxicology studies) or may be suffering from a disorder to be treated with the antibody of interest. The amount of antibody mutant administered may be a therapeutically effective amount to treat the disorder. In dose escalation studies, a variety of doses of the antibody mutant may be administered to the mammal. A therapeutically effective amount of the antibody mutant may be to a human patient to treat a disorder in that patient.

### Brief Description of the Drawings

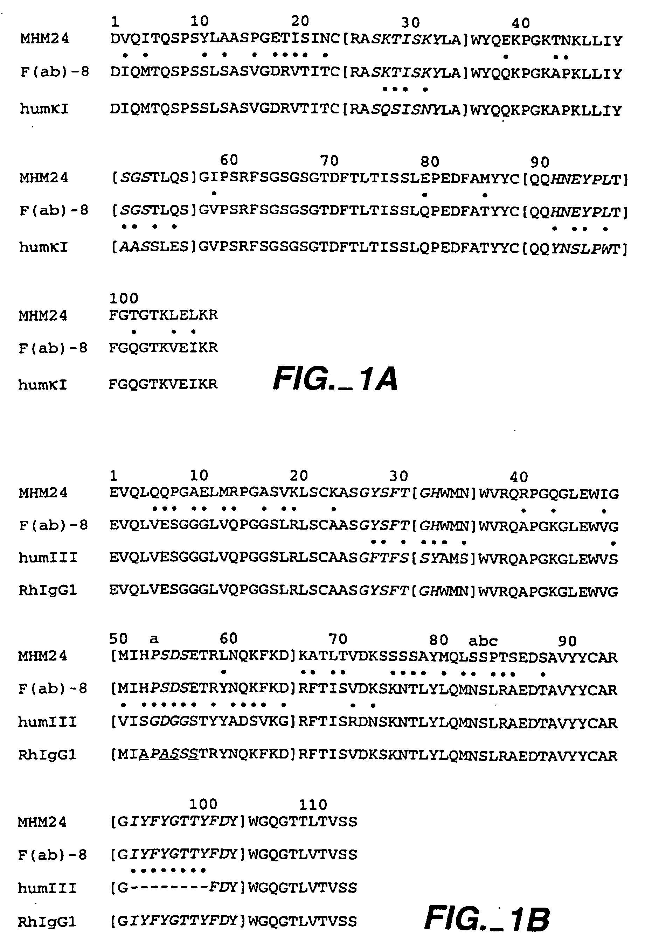

Figure 1A shows the amino acid sequences of murine MHM24 light chain (SEQ ID NO:1), humanized MHM24 F(ab)-8 light chain (SEQ ID NO:2), and human consensus sequence of light chain subgroup κI (humκI) (SEQ ID NO:3).

Figure 1B shows the amino acid sequences of murine MHM24 heavy chain (SEQ ID NO:4), humanized MHM24 F(ab)-8 heavy chain (SEQ ID NO:5), human consensus sequence of heavy chain subgroup III (humIII) (SEQ ID NO:6) and "rhesusized" antibody mutant heavy chain of the Example (RhIgG1) (SEQ ID NO:17).

In Figs. 1A and 1B, hypervariable regions based on sequence hypervariability (Kabat *et al*., *Sequences of Proteins of Immunological Interest.* 5th Ed. Public Health Service, National Institutes of Health. Bethesda, MD. (1991)) are enclosed within brackets and hypervariable loops based on structure of F(ab)-antigen complexes (Chothia *et al*., *Nature* 342:8767 (1989)) are in italics. Residue numbering is according to Kabat *et al*., with insertions shown as a, b, and c.

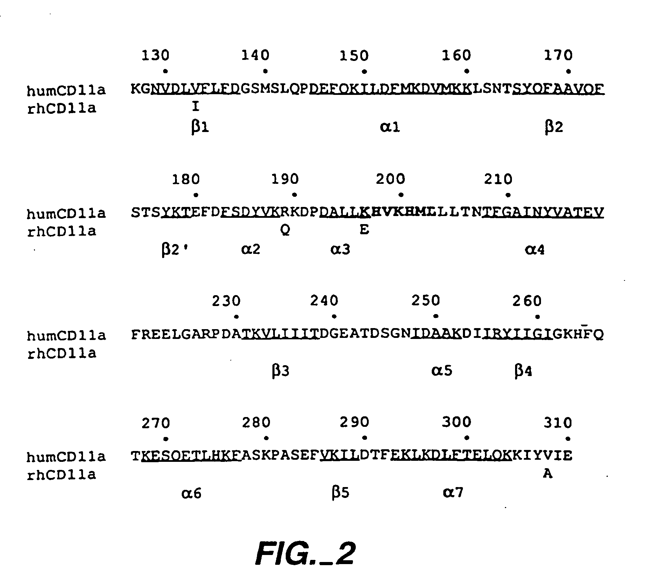

Figure 2 shows sequences of human CD11a 1-domain (SEQ ID NO:7) and rhesus CD11a 1-domain (SEQ ID NO:8). β-strands and α-helices are underlined and labeled according to Qu *et al*., *Proc. Natl*. *Acad Sci*. 92:10277-10281 (1995). The rhesus I-domain sequence (rhCD11a) shows only the four differences from human I-domain. The binding epitope for the MHM24 MAb (SEQ ID NO:9) is shown in bold (Champe *et al*., *J*. *Biol*. *Chem.* 270:1388-1394 (1995)).

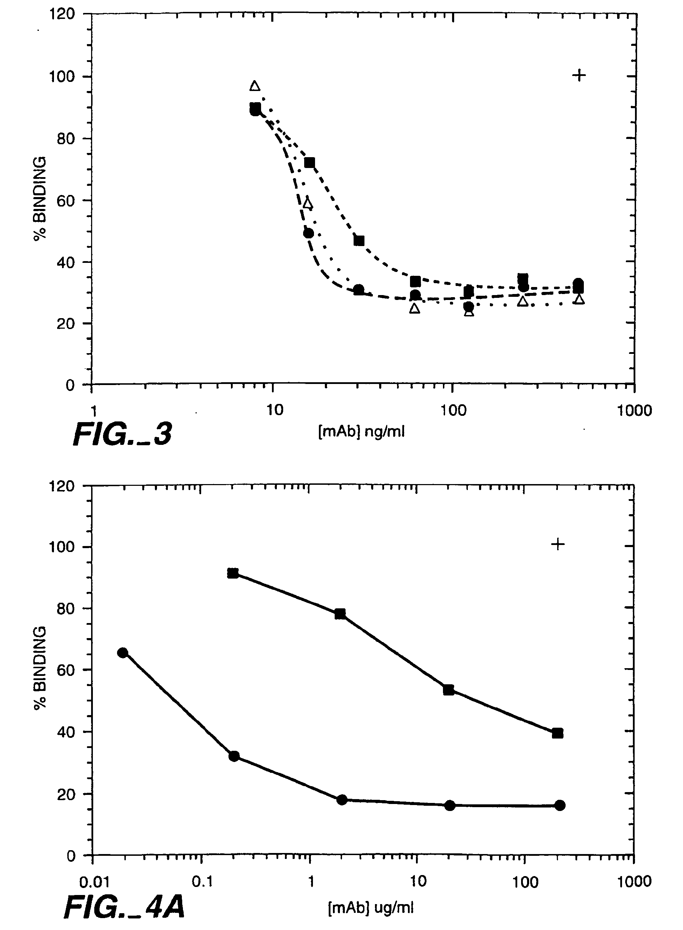

Figure 3 depicts inhibition of human Jurkat T-cells to normal human keratinocytes by murine MHM24 (filled circles), chimeric MHM24 (open triangles), humanized MHM24 (HuIgG1) (filled squares), and a human IgG1 isotype control (+). Percent binding measured by fluorescence of labeled Jurkat cells.

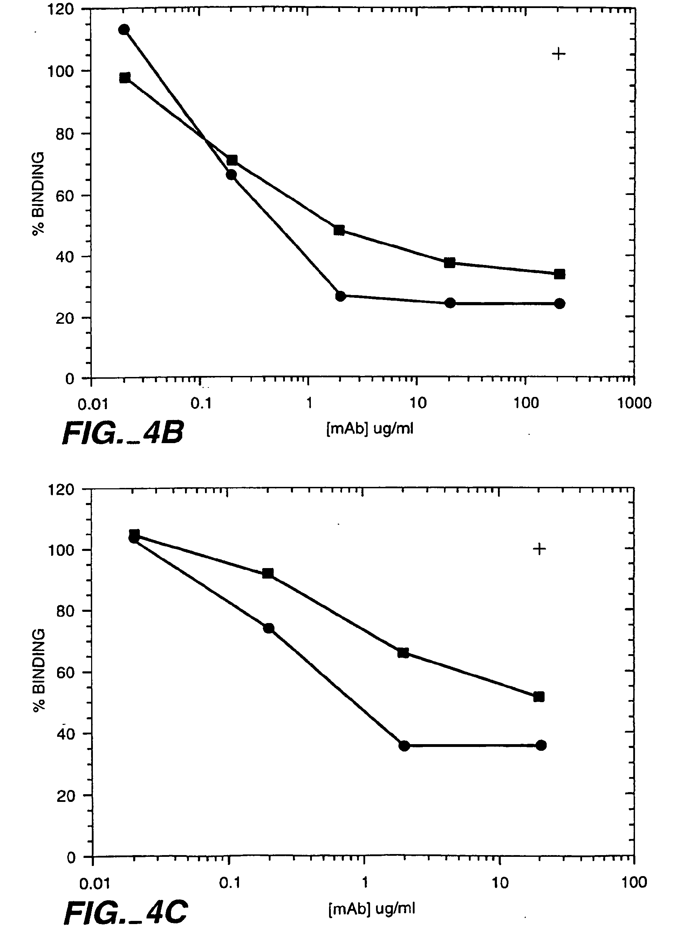

Figures 4A-4C show inhibition of binding of rhesus lymphocytes to normal human keratinocytes (Fig. 4A), rhesus lymphocytes to recombinant human ICAM-1 coated on plates (Fig. 4B), and rhesus/human CD11a chimera-transfected 293 cells to normal human keratinocytes (Fig. 4C). Inhibition by rhesus-binding MHM24 (RhIgG1; filled squares), anti-CD18 MHM23 (filled circles), a human IgG 1 isotype control (+) (Figs. 4A and 4C), and a murine IgG I isotype control (+) (Fig. 4B). Percent binding measured by fluorescence of labeled lymphocytes (Figs. 4A and B) or labeled 293 cells (Fig. 4C).

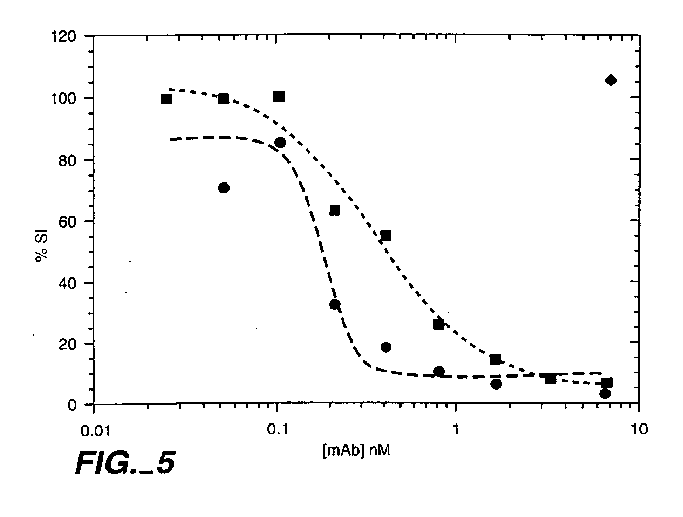

Figure 5 shows human mixed lymphocyte response assy (MLR) is blocked by murine MHM24 (filled circles), humanized MHM24 (HulgG1) (filled squares), and a humanized isotype IgG1 control (filled diamond). Percent stimulation index (%SI) is the ratio of the response at a given MAb concentration to the maximal response with no MAb present. Data is representative of multiple assays using at least two different stimulator/responder pairs.

### Detailed Description of the Preferred Embodiments

### I. Definitions

The term "antibody" is used in the broadest sense and specifically covers monoclonal antibodies (including full length monoclonal antibodies), polyclonal antibodies, multispecific antibodies (*e.g.*, bispecific antibodies), and antibody fragments so long as they exhibit the desired biological activity.

The term "hypervariable region" when used herein refers to the amino acid residues of an antibody which are responsible for antigen-binding. The hypervariable region comprises amino acid residues from a "complementarity determining region" or "CDR" *(i.e.* residues 24-34 (L1), 50-56 (L2) and 89-97 (L3) in the light chain variable domain and 31-35 (H1), 50-65 (H2) and 95-102 (H3) in the heavy chain variable domain; Kabat *et al*., *Sequences of Proteins of Immunological Interest,* 5th Ed. Public Health Service, National Institutes of Health, Bethesda, MD. (1991)) and/or those residues from a "hypervariable loop" (ie. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. *Mol. Biol.* 196:901-917 (1987)). "Framework" or "FR" residues are those variable domain residues other than the hypervariable region residues as herein defined.

"Antibody fragments" comprise a portion of a full length antibody, generally the antigen binding or variable region thereof. Examples of antibody fragments include Fab, Fab', F(ab')₂, and Fv fragments; diabodies; linear antibodies; single-chain antibody molecules; and multispecific antibodies formed from antibody fragments.

The term "monoclonal antibody" as used herein refers to an antibody obtained from a population of substantially homogeneous antibodies, *i*.*e*., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler *et al*., *Nature* 256:495 (1975), or may be made by recombinant DNA methods (see, *e*.*g*., U.S. Patent No. 4,816,567). The "monoclonal antibodies" may also be isolated from phage antibody libraries using the techniques described in Clackson *et al*., *Nature* 352:624-628 (1991) and Marks *et al*., *J*. *Mol. Biol.* 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison *et al., Proc. Natl. Acad. Sci. USA* 81:6851-6855 (1984)).

"Humanized" forms of non-human (*e*.*g*., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones *et al., Nature* 321:522-525 (1986); Riechmann *et al., Nature* 332:323-329 (1988); and Presta, *Curr. Op. Struct. Biol.* 2:593-596 (1992).

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Pluckthun in *The Pharmacology of Monoclonal Antibodies,* vol. 113. Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger *et al., Proc. Natl*. *Acad Sci. USA* 90:6444-6448 (1993).

The expression "linear antibodies" when used throughout this application refers to the antibodies described in Zapata *et al. Protein Eng.* 8(10):1057-1062 (1995). Briefly, these antibodies comprise a pair of tandem Fd segments (V_{H}-C_{H}¹-V_{H}-C_{H}¹) which form a pair of antigen binding regions. Linear antibodies can be bispecific or monospecific.

A "species-dependent antibody" is one which has a stronger binding affinity for an antigen from a first mammalian species than it has for a homologue of that antigen from a second mammalian species. Normally, the species-dependent antibody "binds specifically" to a human antigen (*i*.*e*. has a binding affinity (K_{d}) value of no more than about 1 x 10⁻⁷ M, preferably no more than about 1 x 10⁻⁸ M and most preferably no more than about 1 x 10⁻⁹ M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but preferably is a humanized or human antibody.

As used herein, "antibody mutant" refers to an amino acid sequence variant of the species-dependent antibody wherein one or more of the amino acid residues of the species-dependent antibody have been modified. Such mutants necessarily have less than 100% sequence identity or similarity with the species-dependent antibody. In a preferred embodiment, the antibody mutant will have an amino acid sequence having at least 75% amino acid sequence identity or similarity with the amino acid sequence of either the heavy or light chain variable domain of the species-dependent antibody, more preferably at least 80%, more preferably at least 85%, more preferably at least 90%, and most preferably at least 95%. Identity or similarity with respect to this sequence is defined herein as the percentage of amino acid residues in the candidate sequence that are identical (*i*.*e* same residue) or similar (*i*.*e*. amino acid residue from the same group based on common side-chain properties, see below) with the species-dependent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

"Treatment" refers to both therapeutic treatment and prophylactic or preventative measures. Those in need of treatment include those already with the disorder as well as those in which the disorder is to be prevented.

A "disorder" is any condition that would benefit from treatment with the antibody mutant. This includes chronic and acute disorders or diseases including those pathological conditions which predispose the mammal to the disorder in question. Where the antibody is directed against LFA-1 (e.g. an anti-CD11a antibody) or ICAM-1, the disorder may be a LFA-1 mediated disorder as defined below.

The term "LFA-1-mediated disorder" refers to a pathological state caused by cell adherence interactions involving the LFA-1 receptor on lymphocytes. Examples of such disorders include T cell inflammatory responses such as inflammatory skin diseases including psoriasis; responses associated with inflammatory bowel disease (such as Crohn's disease and ulcerative colitis); adult respiratory distress syndrome; dermatitis; meningitis; encephalitis; uveitis; allergic conditions such as eczema and asthma; conditions involving infiltration ofT cells and chronic inflammatory responses; skin hypersensitivity reactions (including poison ivy and poison oak); atherosclerosis, leukocyte adhesion deficiency; autoimmune diseases such as rheumatoid arthritis, systemic lupus erythematosus (SLE), diabetes mellitus, multiple sclerosis, Reynaud's syndrome, autoimmune thyroiditis, experimental autoimmune encephalomyelitis, Sjorgen's syndrome, juvenile onset diabetes, and immune responses associated with delayed hypersensitivity mediated by cytokines and T-lymphocytes typically found in tuberculosis, sarcoidosis, polymyositis, granulomatosis and vasculitis; pernicious anemia; chronic obstructive pulmonary disease (COPD); bronchitis; insulinitis; rhinitis; urticaria; glomerulonephritis; diseases involving leukocyte diapedesis; CNS inflammatory disorder; multiple organ injury syndrome secondary to septicaemia or trauma: autoimmune hemolytic anemia; myethemia gravis; antigen-antibody complex mediated diseases; nephrotic syndrome; malignancies (e.g., B-cell malignancies such as chronic lymphocytic leukemia or hairy cell leukemia); all types of transplantations, including graft vs. host or host vs. graft disease; HIV and rhinovirus infection; pulmonary fibrosis; invasion of tumor cells into secondary organs etc.

The term "immunosuppressive agent" as used herein for adjunct therapy refers to substances that act to suppress or mask the immune system of the host into which the graft is being transplanted. This would include substances that suppress cytokine production, downregulate or suppress self-antigen expression, or mask the MHC antigens. Examples of such agents include 2-amino-6-aryl-5-substituted pyrimidines (see U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e.g.*, prednisone, methylprednisolone, and dexamethasone; cytokine or cytokine receptor antagonists including anti- interferon- γ, -β, or -α antibodies; anti-tumor necrosis factor-α antibodies; anti-tumor necrosis factor-β antibodies; anti-interleukin-2 antibodies and anti-IL-2 receptor antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90); streptokinase; TGF-β; streptodornase; RNA or DNA from the host; FK506; RS-61443; deoxyspergualin; rapamycin; T-cell receptor (U.S. Pat. No. 5,114,721); T-cell receptor fragments (Offner *et al*., *Science* 251:430-432 (1991); WO 90/11294; and WO 91/01133); and T cell receptor antibodies (EP 340,109) such as T10B9. These agents are administered at the same time or at separate times from the CD11a antibody, and are used at the same or lesser dosages than as set forth in the art. The preferred adjunct immunosuppressive agent will depend on many factors, including the type of disorder being treated including the type of transplantation being performed, as well as the patient's history, but a general overall preference is that the agent be selected from cyclosporin A, a glucocorticosteroid (most preferably prednisone or methylprednisolone), OKT-3 monoclonal antibody, azathioprine, bromocryptine, heterologous anti-lymphocyte globulin, or a mixture thereof.

Unless indicated otherwise, the term "CD11a" when used herein refers to the alpha subunit of LFA-1 from any mammal, but preferably from a human. The CD11a may be isolated from a natural source of the molecule or may be produced by synthetic means (*e*.*g*., using recombinant DNA technology.) The amino acid sequence for human CD11a is described in EP 362 526B1, for example.

The term "I-domain" of CD11a refers to the region of this molecule delineated in Champe *et al*., *J. Biol. Chem.* 270:1388-1394 (1995) and/or Qu *et al. Proc. Natl. Acad Sci.* 92:10277-10281 (1995). The amino acid sequences of human CD11a I-domain (SEQ ID NO:7) and rhesus CD11a I-domain (SEQ ID NO:8) are depicted in Fig. 2 herein.

The term "epitope MHM24" when used herein, unless indicated otherwise, refers to the region in the I-domain of human CD11a to which the MHM24 antibody (see Example below) binds. This epitope comprises the amino acid sequence of SEQ ID NO:9 and, optionally, other amino acid residues of CD1a and/or CD18.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer.

"Mammal" for purposes of treatment refers to any animal classified as a mammal, including humans, domestic and farm animals, nonhuman primates, and zoo, sports, or pet animals, such as dogs, horses, cats, cows, *etc*.

The term "epitope tagged" when used herein refers to an antibody mutant fused to an "epitope tag". The epitope tag polypeptide has enough residues to provide an epitope against which an antibody thereagainst can be made, yet is short enough such that it does not interfere with activity of the antibody mutant. The epitope tag preferably also is fairly unique so that the antibody thereagainst does not substantially cross-react with other epitopes. Suitable tag polypeptide generally have at least 6 amino acid residues and usually between about 8-50 amino acid residues (preferably between about 9-30 residues). Examples include the flu HA tag polypeptide and its antibody 12CA5 (Field *et al*. *Mol*. *Cell. Biol*. 8:2159-2165 (1988)); the c-myc tag and the 8F9, 3C7, 6E10, G4, B7 and 9E10 antibodies thereagainst (Evan *et al*., *Mol*. *Cell. Biol.* 5(12):3610-3616 (1985)); and the Herpes Simplex virus glycoprotein D (gD) tag and its antibody (Paborsky *et al*., *Protein Engineering* 3(6):547-553 (1990)). In certain embodiments, the epitope tag is a "salvage receptor binding epitope".

As used herein, the term "salvage receptor binding epitope" refers to an epitope of the Fc region of an IgG molecule (*e.g*., IgG₁, IgG₂, IgG₃, or IgG₄) that is responsible for increasing the *in vivo* serum half-life of the IgG molecule.

The term "cytotoxic agent" as used herein refers to a substance that inhibits or prevents the function of cells and/or causes destruction of cells. The term is intended to include radioactive isotopes (*e.g.,* I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include Adriamycin, Doxorubicin, 5-Fluorouracil, Cytosine arabinoside ("Ara-C"), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C. Mitoxantrone, Vincreistine, Vinorelbine, Carboplatin, Teniposide, Daunomycin, Carminomycin, Aminopterin, Dactinomycin, Mitomycins, Esperamicins (see U.S. Pat. No. 4,675,187), Melphalan and other related nitrogen mustards.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g.,* Wilman, "Prodrugs in Cancer Chemotherapy" *Biochemical Society Transactions,* 14, pp. 375-382, 615th Meeting Belfast (1986) and Stella *et al*.*,* "Prodrugs: A Chemical Approach to Targeted Drug Delivery," *Directed Drug Delivery,* Borchardt *et al.,* (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactarn-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

The word "label" when used herein refers to a detectable compound or composition which is conjugated directly or indirectly to the antibody. The label may be itself be detectable (*e*.*g*., radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g. controlled pore glass), polysaccharides (*e*.*g*., agarose), polyacrylamides, polystyrene, polyvinyl alcohol and silicones. In certain embodiments, depending on the context, the solid phase can comprise the well of an assay plate; in others it is a purification column (e.g. an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as the antibody mutants disclosed herein and, optionally, a chemotherapeutic agent) to a mammal. The components of the liposome are commonly arranged in a bilayer formation, similar to the lipid arrangement of biological membranes.

An "isolated" nucleic acid molecule is a nucleic acid molecule that is identified and separated from at least one contaminant nucleic acid molecule with which it is ordinarily associated in the natural source of the antibody nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the antibody where, for example, the nucleic acid molecule is in a chromosomal location different from that of natural cells.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide: a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

### II. Modes for Carrying out the Invention

The invention herein relates to a method for making an antibody mutant. The starting antibody is prepared using techniques available in the art for generating such antibodies. Exemplary methods for generating antibodies are described in more detail in the following sections.

The antibody is directed against an antigen of interest. Preferably, the antigen is a biologically important polypeptide and administration of the antibody to a mammal suffering from a disease or disorder can result in a therapeutic benefit in that mammal. However, antibodies directed against nonpolypeptide antigens (such as tumor-associated glycolipid antigens; see US Patent 5,091, 178) are also contemplated.

. Where the antigen is a polypeptide, it may be a transmembrane molecule (e.g. receptor) or ligand such as a growth factor. Exemplary antigens include molecules such as renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant: a plasminogen activator, such as urokinase or human urine or tissue-type plasminogen activator (t-PA); bombesin; thrombin; hemopoietic growth factor, tumor necrosis factor-alpha and -beta: enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β;platelet-derived growth factor (PDGF); fibroblast growth factor such as aFGF and bFGF; epidermal growth factor (EGF); transforming growth factor (TGF) such as TGF-alpha and TGF-beta, including TGF-β 1, TGF-β2, TGF-β3, TGF-β4, or TGF-β5; insulin-like growth factor-I and -11 (IGF-I and IGF-II); des(1-3)-IGF-I (brain IGF-I), insulin-like growth factor binding proteins; CD proteins such as CD3, CD4, CD8, CD19 and CD20; erythropoietin; osteoinductive factors; immunotoxins; a bone morphogenetic protein (BMP); an interferon such as interferon-alpha, -beta, and -gamma; colony stimulating factors (CSFs), e.g., M-CSF, GM-CSF, and G-CSF; interleukins (ILs), e.g., IL- to IL-10**;** superoxide dismutase; T-cell receptors; surface membrane proteins; decay accelerating factor; viral antigen such as, for example, a portion of the AIDS envelope; transport proteins; homing receptors; addressins; regulatory proteins; integrins such as CD11a, CD11b, CD11c, CD18, an ICAM, VLA-4 and VCAM; a tumor associated antigen such as HER2, HER3 or HER4 receptor; and fragments of any of the above-listed polypeptides.

Preferred molecular targets for antibodies encompassed by the present invention include CD proteins such as CD3, CD4, CD8, CD19, CD20 and CD34; members of the ErbB receptor family such as the EGF receptor, HER2, HER3 or HER4 receptor; cell adhesion molecules such as LFA-1, Macl, pl 50,95, VLA-4, ICAM- 1, VCAM and αν/β3 integrin including either α or β subunits thereof (e.g. anti-CD11a, anti-CD18 or anti-CD11b antibodies); growth factors such as VEGF; IgE; blood group antigens; flk2/flt3 receptor; obesity (OB) receptor; *mpl* receptor; CTLA-4; protein C etc.

The antibody is raised against the antigen derived from a first mammalian species. Preferably the first mammalian species is a human. However, other mammals are contemplated such as farm, pet or zoo animals, e.g. where the antibody is intended to be used to treat such mammals. The antigen from the first mammalian species may be isolated from a natural source thereof for the purposes of generating an antibody thereagainst. However, as noted below, cells comprising the antigen can be used as immunogens for making antibodies. In other embodiments, the antigen is produced recombinantly or made using other synthetic methods.

The antibody selected will normally have a sufficiently strong binding affinity for the antigen. For example, the antibody may bind the antigen from the first mammalian species with a binding affinity (K_{d}) value of no more than about I x 10⁻⁷ M, preferably no more than about I x 10⁻⁸ M and most preferably no more than about 1 × 10⁻⁹ M. Antibody affinities may be determined by saturation binding (e.g. as described for anti-CD11a in the Example below); enzyme-linked immunoabsorbent assay (ELISA); and competition assays (e.g. RIA's), for example.

Also, the antibody may be subjected to other biological activity assays, e.g., in order to evaluate its effectiveness as a therapeutic. Such assays are known in the art and depend on the target antigen and intended use for the antibody. Examples include the keratinocyte monolayer adhesion assay and the mixed lymphocyte response (MLR) assay for CD11a (each described in the Example below); tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062).

To screen for antibodies which bind to a particular epitope on the antigen of interest (*e*.*g*., those which block binding of the MHM24 antibody of the Example to CD11a), a routine cross-blocking assay such as that described in *Antibodies*, *A Laboratory Manual*, Cold Spring Harbor Laboratory, Ed Harlow and David Lane (1988), can be performed. Alternatively, epitope mapping, e.g. as described in Champe *et al., J. Biol. Chem.* 270:1388-1394 (1995), can be performed to determine whether the antibody binds an epitope of interest.

Species-dependence of the antibody is then determined. The binding affinity of the antibody for a homologue of the antigen used to generate the antibody (where the homologue is from the "second mammalian species") is assessed using techniques such as those described above. In preferred embodiments, the second mammalian species is a nonhuman mammal tc which the antibody will be administered in preclinical studies. Accordingly, the second mammalian species may be a nonhuman primate, such as rhesus, cynomolgus, baboon, chimpanzee and macaque. In other embodiment, the second mammalian species may be a rodent, cat or dog, for example. The species-dependent antibody will normally have a binding affinity for the antigen from the second nonhuman mammalian species which is at least about 50 fold, or at least about 500 fold, or at least about 1000 fold, weaker than its binding affinity for the antigen from the first mammalian species. This binding affinity will normally be such that the species-dependent antibody cannot effectively be used for preclinical studies in the second mammalian species.

While the preferred method of the instant invention for determining species-dependence (and for evaluating antibody mutants with improved properties; see below) is to quantify antibody binding affinity, in other embodiments of the invention, one or more biological properties of the species-dependent antibody and antibody mutant are evaluated in addition to, or instead of, binding affinity determinations. Exemplary such biological assays are described above. Such assays are particularly useful where they provide an indication as to the therapeutic effectiveness of the antibody. Normally, though not necessarily, antibodies which show improved properties in such assays, will also have an enhanced binding affinity. Thus, in one embodiment of the invention where the assay of choice is a biological activity assay other than an binding affinity assay, the species-dependent antibody will normally have a "biological activity" using "material" (e.g. antigen, cell, tissue, organ or whole animal) from the second mammalian species which is at least about 50 fold, or at least about 500 fold, or at least about 1000 fold, less effective than its biological activity in a corresponding assay using reagents from the first mammalian species.

The species-dependent antibody is then altered so as to generate an antibody mutant which has a stronger binding affinity for the antigen from the second mammalian species, than the species-dependent antibody. The antibody mutant preferably has a binding affinity for the antigen from the nonhuman mammal which is at least about 10 fold stronger, preferably at least about 20 fold stronger, more preferably at least about 50 fold stronger, and sometimes at least about 100 fold or 200 fold stronger, than the binding affinity of the species-dependent antibody for the antigen. The enhancement in binding affinity desired or required will depend on the initial binding affinity of the species-dependent antibody. Where the assay used is a biological activity assay, the antibody mutant preferably has a biological activity in the assay of choice which is at least about 10 fold better, preferably at least about 20 fold better, more preferably at least about 50 fold better, and sometimes at least about 100 fold or 200 fold better, than the biological activity of the species-dependent antibody in that assay.

To generate the antibody mutant, one or more amino acid alterations (e.g. substitutions) are introduced in one or more of the hypervariable regions of the species-dependent antibody. Alternatively, or in addition, one or more alterations (e.g. substitutions) of framework region residues may be introduced in the species-dependent antibody where these result in an improvement in the binding affinity of the antibody mutant for the antigen from the second mammalian species. Examples of framework region residues to modify include those which non-covalently bind antigen directly (Amit *et al*. *Science* 233:747-753 (1986)); interact with/effect the conformation of a CDR (Chothia *et al*. *J*. *Mol*. *Biol*. 196:901-917 (1987)); and/or participate in the V_{L} - V_{H} interface (EP 239 400B1). In certain embodiments, modification of one or more of such framework region residues results in an enhancement of the binding affinity of the antibody for the antigen from the second mammalian species. For example, from about one to about five framework residues may be altered in this embodiment of the invention. Sometimes, this may be sufficient to yield an antibody mutant suitable for use in preclinical trials, even where none of the hypervariable region residues have been altered. Normally, however, the antibody mutant will comprise additional hypervariable region alteration(s).

The hypervariable region residues which are altered may be changed randomly, especially where the starting binding affinity of the species-dependent antibody for the antigen from the second mammalian species is such that such randomly produced antibody mutants can be readily screened.

One useful procedure for generating such antibody mutants is called "alanine scanning mutagenesis" (Cunningham and Wells *Science* 244:1081-1085 (1989)). Here, one or more of the hypervariable region residue(s) are replaced by alanine or polyalanine residue(s) to affect the interaction of the amino acids with the antigen from the second mammalian species. Those hypervariable region residue(s) demonstrating functional sensitivity to the substitutions then are refined by introducing further or other mutations at or for the sites of substitution. Thus, while the site for introducing an amino acid sequence variation is predetermined, the nature of the mutation *per se* need not be predetermined. The ala-mutants produced this way are screened for their biological activity as described herein.

Another procedure for generating such antibody mutants involves affinity maturation using phage display (Hawkins *et al. J. Mol. Biol.* 254:889-896 (1992) and Lowman *et al. Biochemistry* 30(45):10832-10837 (1991)). Briefly, several hypervariable region sites (*e.g.* 6-7 sites) are mutated to generate all possible amino substitutions at each site. The antibody mutants thus generated are displayed in a monovalent fashion from filamentous phage particles as fusions to the gene III product of M13 packaged within each particle. The phage-displayed mutants are then screened for their biological activity (e.g. binding affinity) as herein disclosed.

The invention also provides a more systematic method for identifying amino acid residues to modify. According to this method, one identifies hypervariable region residues in the species-dependent antibody which are involved in binding the antigen from the first mammalian species and those hypervariable region residues involved in binding a homologue of that antigen from the second mammalian species. To achieve this, an alanine-scan of the hypervariable region residues of the species-dependent antibody can be performed, with each ala-mutant being testing for binding to the antigen from the first and second mammalian species (see Example below). The hypervariable region residues involved in binding the antigen from the first mammalian species (e.g. human), and those involved in binding the homologue of the antigen from the second mammalian species (e.g. nonhuman mammal) are thereby identified. Preferably, those residue(s) significantly involved in binding the antigen from the second mammalian species (e.g. nonhuman mammal), but not the antigen from the first mammalian species (e.g. human), are chosen as candidates for modification. In another embodiment, those residue(s) significantly involved in binding the antigen from both the first and second mammalian species are selected to be modified (see Example below). In yet a further but less preferred embodiment, those residues involved in binding the antigen from the first mammalian species, but not the second mammalian species, are selected for modification. Such modification can involve deletion of the residue or insertion of one or more residues adjacent the residue. However, normally the modification involves substitution of the residue for another amino acid.

Normally one would start with a conservative substitution such as those shown in Table I below under the heading of "preferred substitutions". If such substitutions result in a change in biological activity (e.g. binding affinity), then more substantial changes, denominated "exemplary substitutions" in Table I, or as further described below in reference to amino acid classes, are introduced and the products screened.

**Table I**

| Original Residue | Exemplary Substitutions | Preferred Substitutions |
|---|---|---|
| Ala (A) | val; leu; ile | val |
| Arg (R) | lys; gin; asn | lys |
| Asn (N) | gin; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; | leu |
| | phe; norleucine | |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; | leu |
| | ala; norleucine | |

Even more substantial modifications in the antibodies biological properties are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr, asn, gln;
(3) acidic: asp, glu;
(4) basic: his, lys, arg;
(5) residues that influence chain orientation: gly, pro; and
(6) aromatic: trp, tyr, phe.

Non-conservative substitutions will entail exchanging a member of one of these classes for another class.

In another embodiment, the sites selected for modification are affinity matured using phage display (see above).

Nucleic acid molecules encoding amino acid sequence mutants are prepared by a variety of methods known in the art. These methods include, but are not limited to, oligonucleotide-mediated (or site-directed) mutagenesis, PCR mutagenesis, and cassette mutagenesis of an earlier prepared mutant or a non-mutant version of the species-dependent antibody. The preferred method for making mutants is site directed mutagenesis (see, e.g., Kunkel, *Proc. Natl. Acad. Sci. USA* 82:488 (1985)).

In certain embodiments, the antibody mutant will only have a single hypervariable region residue substituted. In other embodiments, two or more of the hypervariable region residues of the species-dependent antibody will have been substituted, e.g. from about two to about ten hypervariable region substitutions. For example, the CD11a antibody mutant of the example below had four hypervariable region substitutions.

Ordinarily, the antibody mutant with improved biological properties will have an amino acid sequence having at least 75% amino acid sequence identity or similarity with the amino acid sequence of either the heavy or light chain variable domain of the species-dependent antibody, more preferably at least 80%, more preferably at least 85%, more preferably at least 90%, and most preferably at least 95%. Identity or similarity with respect to this sequence is defined herein as the percentage of amino acid residues in the candidate sequence that are identical *(i.e* same residue) or similar *(i.e.* amino acid residue from the same group based on common side-chain properties, see above) with the species-dependent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

Following production of the antibody mutant, the biological activity of that molecule relative to the species-dependent antibody is determined. As noted above, this may involve determining the binding affinity and/or other biological activities of the antibody. In a preferred embodiment of the invention, a panel of antibody mutants are prepared above and are screened for binding affinity for the antigen from the second mammalian species. One or more of the antibody mutants selected from this initial screen are optionally subjected to one or more further biological activity assays to confirm that the antibody mutant(s) with enhanced binding affinity are indeed useful, e.g. for preclinical studies. In preferred embodiments, the antibody mutant retains the ability to bind the antigen from the first mammalian species with a binding affinity similar to the species-dependent antibody. This may be achieved by avoiding altering hypervariable region residues involved in binding the antigen from the first mammalian species. In other embodiments, the antibody mutant may have a significantly altered binding affinity for the antigen from the first mammalian species (e.g. the binding affinity for that antigen is preferably better, but may be worse than the species-dependent antibody).

The antibody mutant(s) so selected may be subjected to further modifications, oftentimes depending on the intended use of the antibody. Such modifications may involve further alteration of the amino acid sequence, fusion to heterologous polypeptide(s) and/or covalent modifications such as those elaborated below. With respect to amino acid sequence alterations, exemplary modifications are elaborated above. For example, any cysteine residue not involved in maintaining the proper conformation of the antibody mutant also may be substituted, generally with serine, to improve the oxidative stability of the molecule and prevent aberrant cross linking. Conversely, cysteine bond(s) may be added to the antibody to improve its stability (particularly where the antibody is an antibody fragment such as an Fv fragment). Another type of amino acid mutant has an altered glycosylation pattern. This may be achieved by deleting one or more carbohydrate moieties found in the antibody, and/or adding one or more glycosylation sites that are not present in the antibody. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Techniques for producing antibodies, which may be species-dependent and therefore require modification according to the techniques elaborated herein, follow:

### A. Antibody Preparation

### (i) Antigen preparation

Soluble antigens or fragments thereof, optionally conjugated to other molecules, can be used as immunogens for generating antibodies. For transmembrane molecules, such as receptors, fragments of these (e.g. the extracellular domain of a receptor) can be used as the immunogen. Alternatively, cells expressing the transmembrane molecule can be used as the immunogen. Such cells can be derived from a natural source (*e*.*g*. cancer cell lines) or may be cells which have been transformed by recombinant techniques to express the transmembrane molecule. Other antigens and forms thereof useful for preparing antibodies will be apparent to those in the art.

### (ii) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g.,* 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (iii) Monoclonal antibodies

Monoclonal antibodies may be made using the hybridoma method first described by Kohler *et al*., *Nature*, 256:495 (1975), or may be made by recombinant DNA methods (U.S. Patent No. 4,816,567).

In the hybridoma method, a mouse or other appropriate host animal, such as a hamster or macaque monkey, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, *Monoclonal Antibodies: Principles and Practice,* pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, *J. Immunol.,* 133:3001 (1984); Brodeur *et al., Monoclonal Antibody Production Techniques and Applications,* pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, *Monoclonal Antibodies: Principles and Practice,* pp.59-103 (Academic Press, 1986)). Suitable culture media for this purpose include, for example, D-MEM or RPMI-1640 medium. In addition, the hybridoma cells may be grown *in* vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as E. *coli* cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

In a further embodiment, antibodies or antibody fragments can be isolated from antibody phage libraries generated using the techniques described in McCafferty *et al*., *Nature,* 348:552-554 (1990). Clackson *et al., Nature,* 352:624-628 (1991) and Marks *et al., J. Mol. Biol.,* 222:581-597 (1991) describe the isolation of murine and human antibodies, respectively, using phage libraries. Subsequent publications describe the production of high affinity (nM range) human antibodies by chain shuffling (Marks *et al., Bio*/*Technology*, 10:779-783 (1992)), as well as combinatorial infection and *in vivo* recombination as a strategy for constructing very large phage libraries (Waterhouse *et al., Nuc. Acids. Res.,* 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy-and light-chain constant domains in place of the homologous murine sequences (U.S. Patent No. 4,816,567; Morrison, *et al., Proc. Natl Acad. Sci. USA,* 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (iv) Humanized and human antibodies

A humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones *et **al.,** Nature,* 321:522-525 (1986); Riechmann *et al., Nature,* 332:323-327 (1988); Verhoeyen *et al,. Science,* 239:1534-1536 (1988)), by substituting rodent CDRs or CDR sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims *et al., J. lmmunol.,* 151:2296 (1993); Chothia *et al.. J. Mol. Biol.,* 196:901 (**1987)).** Another method uses a particular framework derived from the consensus sequence of all human antibodies of a particular subgroup of light or heavy chains. The same framework may be used for several different humanized antibodies (Carter *et al., Proc. Natl. Acad Sci. USA,* 89:4285 (1992); Presta *et al., J. Immnol.,* 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i*.*e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the CDR residues are directly and most substantially involved in influencing antigen binding.

Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.,* Jakobovits *et al*., *Proc. Natl. Acad Sci. USA,* 90:2551 (1993); Jakobovits *et al*., *Nature,* 362:255-258 (1993); Bruggermann *et al*., *Year in Immuno*., 7:33 (1993); and Duchosal *et al. Nature* 355:258 (1992). Human antibodies can also be derived from phage-display libraries (Hoogenboom *et al*., *.J Mol*. *Biol.,* 227:381 (1991); Marks *et al*., *J*. *Mol. Biol*., 222:581-597 (1991); Vaughan *et al. Nature Biotech* 14:309 (1996)).

### (v) Antibody fragments

Various techniques have been developed for the production of antibody fragments. Traditionally, these fragments were derived via proteolytic digestion of intact antibodies (see, *e.g.,* Morimoto *et al*., *Journal of Biochemical and Biophysical Methods* 24:107-117 (1992) and Brennan *et al., Science,* 229:81 (1985)). However, these fragments can now be produced directly by recombinant host cells. For example, the antibody fragments can be isolated from the antibody phage libraries discussed above. Alternatively, Fab'-SH fragments can be directly recovered from *E*. *coli* and chemically coupled to form F(ab')₂ fragments (Carter *et al*., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185.

### (vi) Multispecific antibodies

Multispecific antibodies have binding specificities for at least two different antigens. While such molecules normally will only bind two antigens *(i.e**.*** bispecific antibodies, BsAbs), antibodies with additional specificities such as trispecific antibodies are encompassed by this expression when used herein. Examples of BsAbs include those with one arm directed against a tumor cell antigen and the other arm directed against a cytotoxic trigger molecule such as anti-FcγRI/anti-CD15, anti-p185^{HER2}/FcγRIII (CD16), anti-CD3/anti-malignant B-cell (ID10), anti-CD3/anti-p185^{HER2}, anti-CD3/anti-p97, anti-CD3/anti-renal cell carcinoma, anti-CD3/anti-OVCAR-3, anti-CD3/L-D1 (anti-colon carcinoma), anti-CD3/anti-melanocyte stimulating hormone analog, anti-EGF receptor/anti-CD3, anti-CD3/anti-CAMA 1, **anti-CD3/anti-CD 19,** anti-CD3/MoV 18, anti-neural cell ahesion molecule (NCAM)/anti-CD3, anti-folate binding protein (FBP)/anti-CD3, anti-pan carcinoma associated antigen (AMOC-3)/anti-CD3; BsAbs with one arm which binds specifically to a tumor antigen and one arm which binds to a toxin such as anti-saporin/anti-Id-1, anti-CD22/anti-saporin, anti-CD7/anti-saporin, anti-CD38/anti-saporin, anti-CEA/anti-ricin A chain, anti-interferon-α(IFN-α)/anti-hybridoma idiotype, anti-CEA/anti-vinca alkaloid; BsAbs for converting enzyme activated prodrugs such as anti-CD30/anti-alkaline phosphatase (which catalyzes conversion of mitomycin phosphate prodrug to mitomycin alcohol); BsAbs which can be used as fibrinolytic agents such as anti-fibrin/anti-tissue plasminogen activator (tPA), anti-fibrin/anti-urokinase-type plasminogen activator (uPA); BsAbs for targeting immune complexes to cell surface receptors such as anti-low density lipoprotein (LDL)/anti-Fc receptor (e.g. FcγRI, FcγRII or FcγRIII); BsAbs for use in therapy of infectious diseases such as anti-CD3/anti-herpes simplex virus (HSV), anti-T-cell receptor:CD3 complex/anti-influenza, anti-FcγR/anti-HIV; BsAbs for tumor detection *in vitro* or *in vivo* such as anti-CEA/anti-EOTUBE, anti-CEA/anti-DPTA, anti-p185^{HER2}/anti-hapten; BsAbs as vaccine adjuvants; and BsAbs as diagnostic tools such as anti-rabbit IgG/anti-ferritin, anti-horse radish peroxidase (HRP)/anti-hormone, anti-somatostatin/anti-substance P, anti-HRP/anti-FITC, anti-CEA/anti-β-galactosidase. Examples of trispecific antibodies include anti-CD3/anti-CD4/anti-CD37, anti-CD3/anti-CD5/anti-CD37 and anti-CD3/anti-CD8/anti-CD37. Bispecific antibodies can be prepared as full length antibodies or antibody fragments (e.g. F(ab')₂ bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein *et al*., *Nature*, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker *et al*., *EMBO J*., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh *et al., Methods in Enzymology,* 121:210 (1986).

According to another approach described in WO96/27011, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan *et al., Science,* 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E*. *coli,* which can be chemically coupled to form bispecific antibodies. Shalaby *et al., J. Exp. Med.,* 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny *et al., J. Immunol.,* 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger *et al*., *Proc. Natl*. *Acad Sci. USA,* 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber *et al., J. Immunol.,* 152:5368 (1994).

Antibodies with more than two valencies are contemplated. For example, trispecific antibodies can be prepared. Tutt *et al*. *J. Immunol*. 147: 60 (1991).

### (vii) Effector function engineering

It may be desirable to modify the antibody of the invention with respect to effector function, so as to enhance the effectiveness of the antibody in treating cancer, for example. For example cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron *et al*., *J. Exp Med.* 176:1191-1195 (1992) and Shopes, *B.J. Immunol.* 148:2918-2922 (1992). Homodimeric antibodies with enhanced anti-tumor activity may also be prepared using heterobifunctional cross-linkers as described in Wolff *et al*. *Cancer Research* 53:2560-2565 (1993). Alternatively, an antibody can be engineered which has dual Fc regions and may thereby have enhanced complement lysis and ADCC capabilities. See Stevenson *et al. Anti-Cancer Drug Design* 3:219-230 (1989).

### (viii) Immunoconjugates

The antibody mutants of the invention may also be used in immunoconjugates comprising the antibody described herein conjugated to a cytotoxic agent such as a chemotherapeutic agent, toxin (e.g. an enzymatically active toxin of bacterial, fungal, plant or animal origin, or fragments thereof), or a radioactive isotope (*i*.*e*., a radioconjugate).

Chemotherapeutic agents useful in the generation of such immunoconjugates have been described above. Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeceln A chain, alpha-sarcin, *Aleurites fordii* proteins, dianthin proteins, *Phytolaca americana* proteins (PAPI, PAPU, and PAP-S), momordica charantia inhibitor, curcin, crotin, sapaonaria officinalis inhibitor, gelonin, mitogellin, restrictocin, phenomycin, enomycin and the tricothecenes. A variety of radionuclides are available for the production of radioconjugate antibodies. Examples include ²¹²Bi, ¹³¹I, ¹³¹In, ⁹⁰Y and ¹⁸⁶Re.

Conjugates of the antibody and cytotoxic agent are made using a variety of bifunctional protein coupling agents such as N-succinimidyl-3-(2-pyridyldithiol) propionate (SPDP), iminothiolane (IT). bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine),bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro 2,4-dinitrobenzene). For example, a ricin immunotoxin can be prepared as described in Vitetta *et al. Science* 238: 1098 (1987). Carbon-14-labeled 1-isothiocyanatobenzyl-3-methyldiedthylene triaminepentaacetic acid (MX-DTPA) is an exemplary chelating agent for conjugation of radionucleotide to the antibody. See WO94/11026.

In another embodiment, The antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

### (α) Immunoliposomes

The antibody mutants disclosed herein may also be formulated as immunoliposomes. Liposomes containing the antibody are prepared by methods known in the art, such as described in Epstein *et al*., *Proc. Natl*. *Acad Sci* USA, 82:3688 (1985); Hwang *et al., Proc. Natl Acad Sci. USA*, 77:4030 (1980); and U.S. Pat. Nos. 4,485,045 and 4,544,545. Liposomes with enhanced circulation time are disclosed in US. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent (such as Doxorubicin) is optionally contained within the liposome. See Gabizon *et al*. *J*. *National Cancer Inst*.81(19)1484 (1989)

### (x) Antibody Dependent Enzyme Mediated Prodrug Therapy (ADEPT)

The antibody of the present invention may also be used in ADEPT by conjugating the antibody to a prodrug-activating enzyme which converts a prodrug (e.g. a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents: carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, *Nature* 328: 457-458 (1987)). Antibody-abzyme conjugates can be prepared as described herein for delivery of the abzyme to a tumor cell population.

The enzymes of this invention can be covalently bound to the antibody mutant by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, e.g., Neuberger *et al., Nature,* 312: 604-608 (1984)).

### (xi) Antibody-salvage receptor binding epitope fusions.

In certain embodiments of the invention, it may be desirable to use an antibody fragment, rather than an intact antibody, to increase tumor penetration, for example. In this case, it may be desirable to modify the antibody fragment in order to increase its serum half life. This may be achieved, for example, by incorporation of a salvage receptor binding epitope into the antibody fragment (*e.g.* by mutation of the appropriate region in the antibody fragment or by incorporating the epitope into a peptide tag that is then fused to the antibody fragment at either end or in the middle, e.g., by DNA or peptide synthesis).

The salvage receptor binding epitope preferably constitutes a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (e.g., of an IgG) and transferred to the CH1, CH3, or V_{H} region, or more than one such region, of the antibody. Alternatively, the epitope is taken from the CH2 domain of the Fc region and transferred to the C_{L} region or V_{L} region, or both, of the antibody fragment.

In one most preferred embodiment, the salvage receptor binding epitope comprises the sequence (5' to 3'): PKNSSMISNTP (SEQ ID NO:10), and optionally further comprises a sequence selected from the group consisting of HQSLGTQ (SEQ ID NO:11), HQNLSDGK (SEQ ID NO:12), HQNISDGK (SEQ ID NO:13), or VISSHLGQ (SEQ ID NO:14), particularly where the antibody fragment is a Fab or F(ab')₂. In another most preferred embodiment, the salvage receptor binding epitope is a polypeptide containing the sequence(s)(5' to 3'): HQNLSDGK (SEQ ID NO:12), HQNISDGK (SEQ ID NO:13), or VISSHLGQ (SEQ ID NO:14) and the sequence: PKNSSMISNTP (SEQ ID NO:10).

### (xii) Other covalent modifications of the antibody

Covalent modifications of the antibody are included within the scope of this invention. They may be made by chemical synthesis or by enzymatic or chemical cleavage of the antibody, if applicable. Other types of covalent modifications of the antibody are introduced into the molecule by reacting targeted amino acid residues of the antibody with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino-terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate, pyridoxal phosphate, pyridoxal, chloroborohydride, trinitrobenzenesulfonic acid, O-methylisourea, 2,4-pentanedione, and transaminase-catalyzed reaction with glyoxylate.

Arginyl residues are modified by reaction with one or several conventional reagents, among them phenylglyoxal, 2,3-butanedione, l,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay.

Carboxyl side groups (aspartyl or glutamyl) are selectively modified by reaction with carbodiimides (R-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of this invention.

Other modifications include hydroxylation of proline and lysine, phosphorylation of hydroxyl groups of seryl or threonyl residues, methylation of the a-amino groups of lysine, arginine, and histidine side chains (T.E. Creighton, *Proteins: Structure and Molecular Properties,* W.H. Freeman & Co., San Francisco; pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 1September 1987, and in Aplin and Wriston, *CRC Crit. Rev. Biochem.,* pp. 259-306 (1981).

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Hakimuddin, *et al. Arch. Biochem. Biophys.* 259:52 (1987) and by Edge *et al*. *Anal. Biochem.,* 118:131 (1981). Enzymatic cleavage of carbohydrate moieties on antibodies can be achieved by the use of a variety of endo- and exo-glycosidases as described by Thotakura *et al. Meth. Enzymol.* 138:350 (1987).

Another type of covalent modification of the antibody comprises linking the antibody to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

### B. Vectors, Host Cells and Recombinant Methods

The invention also provides isolated nucleic acid encoding an antibody mutant as disclosed herein, vectors and host cells comprising the nucleic acid, and recombinant techniques for the production of the antibody mutant.

For recombinant production of the antibody mutant, the nucleic acid encoding it is isolated and inserted into a replicable vector for further cloning (amplification of the DNA) or for expression. DNA encoding the monoclonal antibody mutant is readily isolated and sequenced using conventional procedures (e.g., by using oligonucleotide probes that are capable of binding specifically to genes encoding the heavy and light chains of the antibody mutant). Many vectors are available. The vector components generally include, but are not limited to, one or more of the following: a signal sequence, an origin of replication, one or more marker genes, an enhancer element, a promoter, and a transcription termination sequence.

### (i) Signal sequence component

The antibody mutant of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed *(i.e.,* cleaved by a signal peptidase) by the host cell. For prokaryotic host cells that do not recognize and process the native antibody signal sequence, the signal sequence is substituted by a prokaryotic signal sequence selected, for example, from the group of the alkaline phosphatase, penicillinase, lpp, or heat-stable enterotoxin II leaders. For yeast secretion the native signal sequence may be substituted by, e.g., the yeast invertase leader, α factor leader (including *Saccharomyces* and *Kluyveromyces* α-factor leaders), or acid phosphatase leader, the C. *albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the antibody mutant.

### (ii) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### (iii) Selection gene component

Expression and cloning vectors may contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, *etc*.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding antibody, wild-type DHFR protein, and another selectable marker such as aminoglycoside 3'-phosphotransferase (APH) can be selected by cell growth in medium containing a selection agent for the selectable marker such as an aminoglycosidic antibiotic, *e.g.,* kanamycin, neomycin, or G418. See U.S. Patent No. 4,965,199.

A suitable selection gene for use in yeast is the *trp*1 gene present in the yeast plasmid YRp7 (Stinchcomb *et al., Nature,* 282:39 (1979)). The *trp*1 gene provides a selection marker for a mutant strain of yeast lacking the ability to grow in tryptophan, for example, ATCC No. 44076 or PEP4-1. Jones, *Genetics,* 85:12 (1977). The presence of the *trp1* lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

In addition, vectors derived from the 1.6 µm circular plasmid pKD1 can be used for transformation *of Kluyveromyces* yeasts. Alternatively, an expression system for large-scale production.of recombinant calf chymosin was reported for *K. lactis.* Van den Berg, *Bio*/*Technology*, 8:135 (1990). Stable multi-copy expression vectors for secretion of mature recombinant human serum albumin by industrial strains of *Kluyveromyces* have also been disclosed. Fleer *et al*., *Bio*/*Technology*, 9:968-975 (1991).

### (iv) Promoter component

Expression and cloning vectors usually contain a promoter that is recognized by the host organism and is operably linked to the antibody nucleic acid. Promoters suitable for use with prokaryotic hosts include the *phoA* promoter , β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes *et al*., *Nature* 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### (v) Enhancer element component

Transcription of a DNA encoding the antibody of this invention by higher eukaryotes is often increased by inserting an enhancer sequence into the vector. Many enhancer sequences are now known from mammalian genes (globin, elastase, albumin, a-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, *Nature* 297:17-18 (1982) on enhancing elements for activation of eukaryotic promoters. The enhancer may be spliced into the vector at a position 5' or 3' to the antibody-encoding sequence, but is preferably located at a site 5' from the promoter.

### (vi) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the antibody. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia*, *e.g., E. coli, Enterobacter, Erwinia, Klebsiella, Proteus, Salmonella, e.g., Salmonella typhimurium*, *Serratia, e.g., Serratia marcescans,* and *Shigella,* as well as *Bacilli* such as *B. subtilis* and *B. licheniformis (e.g., B*. *licheniformis* 41P disclosed in DD 266,710 published 12 April 1989), *Pseudomonas* such as *P. aeruginosa,* and *Streptomyces.* One preferred *E. coli* cloning host is *E*. *coli* 294 (ATCC 31,446), although other strains such as *E*. *coli* B, E. *coli* X1776 (ATCC 31,537), and *E*. *coli* W3110 (ATCC 27,325) are suitable. These examples are illustrative rather than limiting.

In addition to prokaryotes, eukaryotic microbes such as filamentous fungi or yeast are suitable cloning or expression hosts for antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e.g., K. lactis. K fragilis* (ATCC 12,424), K. *bulgaricus* (ATCC 16,045), K. *wickeramii* (ATCC 24,178), *K. waltii* (ATCC 56,500), K *drosophilarum* (ATCC 36,906), *K. thermotolerans,* and *K*. *marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, *e.g., Neurospora, Penicillium, Tolypocladium,* and *Aspergillus* hosts such as *A. nidulans* and *A*. *niger.*

Suitable host cells for the expression of glycosylated antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, *e*.*g*., the L-I variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al., J. Gen Virol.* 36:59 (1977)); baby hamster kidney cells (BHK, ATCC CCL 10); Chinese hamster ovary cells/-DHFR (CHO, Urlaub *et al., Proc. Natl*. *Acad Sci. USA* 77:4216 (1980)); mouse sertoli cells (TM4, Mather, *Biol. Reprod* 23:243-251 (1980)); monkey kidney cells (CV 1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al., Annals N. Y*. *Acad Sci.* 383:44-68 (1982)); MRC 5 cells: FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the host cells

The host cells used to produce the antibody mutant of this invention may be cultured in a variety of media. Commercially available media such as Ham's **F10** (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham *et al., Meth. Enz.* 58:44 (1979), Barnes *et al., Anal. Biochem.102:255* (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Antibody purification

When using recombinant techniques, the antibody mutant can be produced intracellularly, in the periplasmic space, or directly secreted into the medium. If the antibody mutant is produced intracellularly, as a first step, the particulate debris, either host cells or lysed fragments, is removed, for example, by centrifugation or ultrafiltration. Carter *et al*., *Bio*/*Technology* 10:163-167 (1992) describe a procedure for isolating antibodies which are secreted to the periplasmic space of *E*. *coli*. Briefly, cell paste is thawed in the presence of sodium acetate (pH 3.5), EDTA, and phenylmethylsulfonylfluoride (PMSF) over about 30 min. Cell debris can be removed by centrifugation. Where the antibody mutant is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody mutant. Protein A can be used to purify antibodies that are based on human γ 1, γ2, or γ4 heavy chains (Lindmark *et al., J. Immunol. Meth.* 62:1-13 (1983)). Protein G is recommended for all mouse isotypes and for human y3 (Guss *et al., EMBO J.* 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody mutant comprises a C_{H}3 domain, the Bakerbond ABX^{TM}resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE^{TM} chromatography on an anion or cation exchange resin (such as a polyaspartic acid column), chromatofocusing, SDS-PAGE, and ammonium sulfate precipitation are also available depending on the antibody mutant to be recovered.

Following any preliminary purification step(s), the mixture comprising the antibody mutant of interest and contaminants may be subjected to low pH hydrophobic interaction chromatography using an elution buffer at a pH between about 2.5-4.5, preferably performed at low salt concentrations (*e.g.*, from about 0-0.25M salt).

### C. Pharmaceutical Formulations

Therapeutic formulations of the antibody mutant are prepared for storage by mixing the antibody mutant having the desired degree of purity with optional physiologically acceptable carriers, excipients or stabilizers *(Remington's Pharmaceutical Sciences* 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol: and m-cresol); low molecular weight (less than about 10 residues) polypeptide; proteins, such as serum albumin, gelatin, or immunoglobulins: hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN^{™}, PLURONICS^{™} or polyethylene glycol (PEG).

The formulation herein may also contain more than one active compound as necessary for the particular indication being treated, preferably those with complementary activities that do not adversely affect each other. For example, it may be desirable to further provide an immunosuppressive agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in *Remington's Pharmaceutical Sciences* 16th edition, Osol, A. Ed. (1980).

The formulations to be used for in *vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody mutant, which matrices are in the form of shaped articles, *e*.*g*., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyt-L-gtutamate, non-degradable ethylene-vinyl acetate, degradable lactic acid-glycolic acid copolymers such as the LUPRON DEPOT^{TM} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37° C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### D. Non-Therapeutic Uses for the Antibody Mutant

The antibody mutants of the invention may be used as affinity purification agents. In this process, the antibodies are immobilized on a solid phase such a Sephadex resin or filter paper, using methods well known in the art. The immobilized antibody mutant is contacted with a sample containing the antigen to be purified, and thereafter the support is washed with a suitable solvent that will remove substantially all the material in the sample except the antigen to be purified, which is bound to the immobilized antibody mutant. Finally, the support is washed with another suitable solvent, such as glycine buffer, pH 5.0, that will release the antigen from the antibody mutant.

The mutant antibodies may also be useful in diagnostic assays, *e*.*g*., for detecting expression of an antigen of interest in specific cells, tissues, or serum.

For diagnostic applications, the antibody mutant typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The antibody mutant can be labeled with the radioisotope using the techniques described in *Current Protocols in Immunology,* Volumes I and 2. Coligen *et al.,* Ed. Wiley-Interscience, New York, New York, Pubs. (1991) for example and radioactivity can be measured using scintillation counting.
(b) Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the antibody mutant using the techniques disclosed in *Current Protocols in Immunology, supra,* for example. Fluorescence can be quantified using a fluorimeter.
(c) Various enzyme-substrate labels are available and U.S. Patent No. 4,275,149 provides a review of some of these. The enzyme generally catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan *et al,.* Methods for the Preparation of Enzyme-Antibody Conjugates for use in Enzyme lmmunoassay, in *Methods in Enzym.* (ed J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase oxidizes a dye precursor (*e.g..*orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (e.g.. p-nitrophenyl-β-D-galactosidase) or fluorogenic substrate 4-methylumbelliferyl-β-D-galactosidase.

Numerous other enzyme-substrate combinations are available to those skilled in the art. For a general review of these, see U.S. Patent Nos. 4,275,149 and 4,318,980.

Sometimes, the label is indirectly conjugated with the antibody mutant. The skilled artisan will be aware of various techniques for achieving this. For example, the antibody mutant can be conjugated with biotin and any of the three broad categories of labels mentioned above can be conjugated with avidin, or *vice versa.* Biotin binds selectively to avidin and thus, the label can be conjugated with the antibody mutant in this indirect manner. Alternatively, to achieve indirect conjugation of the label with the antibody mutant, the antibody mutant is conjugated with a small hapten (*e*.*g*., digoxin) and one of the different types of labels mentioned above is conjugated with an anti-hapten antibody mutant (e.g., anti-digoxin antibody). Thus, indirect conjugation of the label with the antibody mutant can be achieved.

In another embodiment of the invention, the antibody mutant need not be labeled, and the presence thereof can be detected using a labeled antibody which binds to the antibody mutant.

The antibodies of the present invention may be employed in any known assay method, such as competitive binding assays, direct and indirect sandwich assays, and immunoprecipitation assays. Zola, *Monoclonal Antibodies: A Manual of Techniques,* pp. 147-158 (CRC Press, Inc. 1987).

Competitive binding assays rely on the ability of a labeled standard to compete with the test sample analyze for binding with a limited amount of antibody mutant. The amount of antigen in the test sample is inversely proportional to the amount of standard that becomes bound to the antibodies. To facilitate determining the amount of standard that becomes bound, the antibodies generally are insolubilized before or after the competition, so that the standard and analyze that are bound to the antibodies may conveniently be separated from the standard and analyze which remain unbound.

Sandwich assays involve the use of two antibodies, each capable of binding to a different immunogenic portion, or epitope, of the protein to be detected. In a sandwich assay, the test sample analyze is bound by a first antibody which is immobilized on a solid support, and thereafter a second antibody binds to the analyze, thus forming an insoluble three-part complex. See, e.g.. US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tumor sample may be fresh or frozen or may be embedded in paraffin and fixed with a preservative such as formalin, for example.

The antibodies may also be used for *in vivo* diagnostic assays. Generally, the antibody mutant is labeled with a radionuclide (such as ¹¹¹In, ⁹⁹Tc, ¹⁴C, ¹³¹I, ¹²⁵I, ³H, ³²P or ³⁵S) so that the tumor can be localized using immunoscintiography.

### E. Diagnostic Kits

As a matter of convenience, the antibody mutant of the present invention can be provided in a kit, *i*.*e*., a packaged combination of reagents in predetermined amounts with instructions for performing the diagnostic assay. Where the antibody mutant is labeled with an enzyme, the kit will include substrates and cofactors required by the enzyme *(e.g.,* a substrate precursor which provides the detectable chromophore or fluorophore). In addition, other additives may be included such as stabilizers, buffers *(e.g.,* a block buffer or lysis buffer) and the like. The relative amounts of the various reagents may be varied widely to provide for concentrations in solution of the reagents which substantially optimize the sensitivity of the assay. Particularly, the reagents may be provided as dry powders, usually lyophilized, including excipients which on dissolution will provide a reagent solution having the appropriate concentration.

### F. In Vivo Uses for the Antibody Mutant

It is contemplated that the antibody mutant of the present invention may be used to treat a mammal. In one embodiment, the antibody is administered to a nonhuman mammal for the purposes of obtaining preclinical data, for example. Exemplary nonhuman mammals to be treated include nonhuman primates, dogs, cats, rodents and other mammals in which preclinical studies are performed. Such mammals may be established animal models for a disease to be treated with the antibody or may be used to study toxicity of the antibody of interest. In each of these embodiments, dose escalation studies may be performed in the mammal. Where the antibody is an anti-CD11a antibody, it may be administered to a rhesus monkey in a heart allograft model, for example.

In addition, or in the alternative, the antibody mutant is used to treat a human, e.g. a patient suffering from a disease or disorder who could benefit from administration of the antibody mutant. The conditions which can be treated with the antibody are many and include LFA-mediated disorders (e.g. where the antibody is an anti-LFA-1 or anti-ICAM-1 antibody); cancer *(e.g.* where the antibody binds the HER2 receptor or CD20); allergic conditions such as asthma (with an anti-IgE antibody) etc.

The antibody mutant is administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the antibody mutant is suitably administered by pulse infusion, particularly with declining doses of the antibody mutant. Preferably the dosing is given by injections, most preferably intravenous or subcutaneous injections, depending in pan on whether the administration is brief or chronic.

For the prevention or treatment of disease, the appropriate dosage of antibody mutant will depend on the type of disease to be treated, the severity and course of the disease, whether the antibody mutant is administered for preventive or therapeutic purposes, previous therapy, the patient's clinical history and response to the antibody mutant, and the discretion of the attending physician. The antibody mutant is suitably administered to the patient at one time or over a series of treatments

Depending on the type and severity of the disease, about 1 µg/kg to 15 mg/kg (e.g 0.1-20mg/kg) of antibody mutant is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. An exemplary dosing regimen for an anti-LFA-I or anti-ICAM-I antibody is disclosed in WO 94/04188.

The antibody mutant composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody mutant to be administered will be governed by such considerations, and is the minimum amount necessary to prevent, ameliorate, or treat a disease or disorder. The antibody mutant need not be, but is optionally formulated with one or more agents currently used to prevent or treat the disorder in question. The effective amount of such other agents depends on the amount of antibody mutant present in the formulation, the type of disorder or treatment, and other factors discussed above. These are generally used in the same dosages and with administration routes as used hereinbefore or about from 1 to 99% of the heretofore employed dosages.

### G. Articles of Manufacture

The mutant antibody of the invention may be used in an article of manufacture containing materials useful for the treatment of the disorders described above The article of manufacture comprises a container and a label Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the antibody mutant. The label on, or associated with, the container indicates that the composition is used for treating the condition of choice. The article of manufacture may further comprise a second container comprising a pharmaceutically-acceptable buffer, such as phosphate-buffered saline. Ringer's solution and dextrose solution. It may further include other materials desirable from a commercial and user standpoint, including other buffers, diluents, filters, needles, syringes, and package inserts with instructions for use.

### EXAMPLE

### PRODUCTION OF ANTIBODY MUTANTS

This example describes the humanization and *in vitro* biological efficacy of a murine anti-human CD1 la monoclonal antibody, MHM24 (Hildreth *et al., Eur. J. Immunol.* 13:202-208 (1983)). Previous studies on the murine MHM24 have shown that it, like other anti-CD1 la antibodies, can inhibit T cell function (Hildreth *et al., J. lmmunol.* 134:3272-3280 (1985); Dougherty *et al., Eur. **J.** Immunol.* 17:943-947 (1987)). Both the murine and humanized MAbs effectively prevent adhesion of human T cells to human keratinocytes and the proliferation of T cells in response to nonautologous leukocytes in the mixed lymphocytes response (MLR), a model for responsiveness to MHC class II antigens (McCabe *et al., Cellular Immunol.* 150:364-375 (1993)). However, both the murine (Reimann *et al., Cytometry,* 17:102-108 (1994)) and humanized MAbs did not cross-react with nonhuman primate CD11a other than chimpanzee CD11a. In order to have a humanized MAb available for preclinical studies in rhesus, the humanized MAb was re-engineered to bind to rhesus CD11a by changing four residues in one of the complementarity-determining regions, CDR-H2, in the variable heavy domain. Cloning and molecular modeling of the rhesus CD11a I-domain suggested that a change from a lysine residue in human CD11a I-domain to glutamic acid in rhesus CD11a I-domain is the reason that the murine and humanized MAbs do not bind rhesus CD11a.

### Materials and Methods

### (a) Construction of humanized F(ab')s

The murine anti-human CD11a MAb, MHM24 (Hildreth *et al*., *Eur. J*. *Immunol*. 13:202-208 (1983); Hildreth *et al*., *J. Immunol*. 134:3272-3280 (1985)), was cloned and sequenced. In order to have a plasmid useful for mutagenesis as well as for expression of F(ab)s in *E. coli,* the phagemid pEMX I was constructed. Based on the phagemid pb0720, a derivative of pB0475 (Cunningham *et al*., *Science* 243:1330-1336 (1989)), pEMX1 contains a DNA fragment encoding a humanized κ-subgroup I light chain and a humanized subgroup III heavy chain (VH-CH1) and an alkaline phosphatase promotor and Shine-Dalgamo sequence both derived from another previously described pUC119-based plasmid, pAK2 (Carter *et al*., *Proc. Natl*. *Acad Sci. USA* 89:4285 (1992)). A unique Spel restriction site was also introduced between the DNA encoding for the F(ab) light and heavy chains.

To construct the first F(ab) mutant of humanized MHM24, F(ab)-1, site-directed mutagenesis (Kunkel, *Proc. Natl. Acad Sci. USA* 82:488 (1985)) was performed on a deoxyuridine-containing template of pEMX1; all six CDRs were changed to the MHM24 sequence. All other F(ab) mutants were constructed from a template of F(ab)-1. Plasmids were transformed into *E. coli* strain XL-1 Blue (Stratagene, San Diego, CA) for preparation of double- and single-stranded DNA. For each mutant both light and heavy chains were completely sequenced using the dideoxynucleotide method (Sequenase, U.S. Biochemical Corp.). Plasmids were transformed into *E. coli* strain 16C9, a derivative of MM294, plated onto LB plates containing 5 µg/ml carbenicillin, and a single colony selected for protein expression. The single colony was grown in 5 ml LB-100 µg/ml carbenicillin for 5-8 h at 37°C. The 5 ml culture was added to 500 ml AP5-100 µg/ml carbenicillin and allowed to grow for 16 h in a 4 L baffled shake flask at 37° C. APS media consists of: 1.5 g glucose, 11.0 Hycase SF, 0.6 g yeast extract (certified), 0.19 g MgSO4 (anhydrous), 1.07 g NH4Cl, 3.73 g KCI, 1.2 g NaCl, 120 ml 1 M triethanolamine, pH 7.4, to 1 L water and then sterile filtered through 0.1 µm Sealkeen filter.

Cells were harvested by centrifugation in a 1L centrifuge bottle (Nalgene) at 3000 x g and the supernatant removed. After freezing for I h, the pellet was resuspended in 25 ml cold 10 mM MES-10 mM EDTA, pH 5.0 (buffer A). 250 µl of 0.1 M PMSF (Sigma) was added to inhibit proteolysis and 3.5 ml of stock 10 mg/ml hen egg white lysozyme (Sigma) was added to aid lysis of the bacterial cell wall. After gentle shaking on ice for 1h, the sample was centrifuged at 40,000 x g for 15 min. The supernatant was brought to 50 ml with buffer A and loaded onto a 2 ml DEAE column equilibrated with buffer A. The flow-through was then applied to a protein G-Sepharose CL-4B (Pharmacia) column (0.5 ml bed volume) equilibrated with buffer A. The column was washed with 10 ml buffer A and eluted with 3 ml 0.3 M glycine, pH 3.0, into 1.25 ml I M Tris, pH 8.0. The F(ab) was then buffer exchanged into PBS using a Centricon-30 (Amicon) and concentrated to a final volume of 0.5 ml. SDS-PAGE gels of all F(ab)s were run to ascertain purity and the molecular weight of each mutant was verified by electrospray mass spectrometry.

### (b) Construction of chimeric and humanized lgG

For generation of human IgG1 mutants of chimeric (chlgG1) and humanized (HulgG1) MHM24, the appropriate murine or humanized variable light and variable heavy (F(ab)-8, Table II) domains were subcloned into separate previously described pRK vectors (Gorman *et al., DNA Protein Eng. Tech.* 2:3 (1990)). Alanine-scan mutants were constructed by site-directed mutagenesis (Kunkel. *Proc. Natl. Acad. Sci. USA* 82:488 (1985)) of the HuIgG1 light and heavy chain plasmids. The DNA sequence of each mutant was verified by dideoxynucleotide sequencing.

Heavy and light chain plasmids were cotransfected into an adenovirus-transformed human embryonic kidney cell line, 293 (Graham *et al., J. Gen. Virol.* 36:59 (1977)), using a high efficiency procedure (Graham *et al., J. Gen. Virol.* 36:59 (1977); Gorman *et al., Science,* 221:551 (1983)). Media was changed to serum-free and harvested daily for up to 5 days. Antibodies were purified from the pooled supernatants using protein A-Sepharose CL-4B (Pharmacia). The eluted antibody was buffer exchanged into PBS using a Centricon-30 (Amicon), concentrated to 0.5 ml, sterile filtered using a Millex-GV (Millipore) and stored at 4°C.

The concentration of antibody was determined using total Ig-binding ELISA. The concentration of the reference humanized anti-p 185^{HER}IgG1 (Carter *et al., Proc. Natl. Acad Sci. USA* 89:4285 (1992)) was determined by amino acid composition analysis. Each well of a 96-well plate was coated with 1 µg/ml of goat anti-human IgG F(ab')₂ (Cappel Laboratories, Westchester, PA) for 24 h at 4°C. Purified antibodies were diluted and added in duplicate to the coated plates. After 1.5 h incubation, the plates were washed with PBS-0.02% Tween 20 and 0.1 1 ml of a 1:2000 dilution of horseradish peroxidase-conjugated sheep anti-human IgG F(ab')₂ (Cappel) was added. After 1.5 h incubation the plates were washed and 0.1 ml 0.2 mg/ml o-phenylenediamine dihydrochloride-0.01% hydrogen peroxide-PBS was added. After 10 min. the reaction was stopped with 2 M sulfuric acid and the O.D. 490 nm was read.

### (c) Cloning of rhesus CD11a I-domain

The DNA sequence of the rhesus I-domain was obtained using RT-PCR and primers derived from the human CD11a DNA sequence. Briefly, mRNA was isolated from ~10⁷ rhesus leukocytes using the Fast Track mRNA purification kit (Invitrogen). 10 µg mRNA was reverse transcribed using MuLV reverse transcriptase. The first strand cDNA was then amplified by 40 cycles of PCR using the primers:
5'-CACTTTGGATACCGCGTCCTGCAGGT-3' (forward)(SEQ ID NO:15) and
5'-CATCCTGCAGGTCTGCCTTCAGGTCA-3' (reverse)(SEQ ID NO:16).

A single band of the predicted size was purified from the PCR reaction using agarose gel electrophoresis. The PCR product was digested with restriction enzyme Sse83871 (Takara) and ligated to a human CD11a -containing plasmid digested with the same restriction enzyme. There are two Sse83871 sites in the human CD11a sequence, one on either side of the 1-domain. The resulting plasmid encoded a chimera consisting of human CD11a with a rhesus I-domain substituted for the human I-domain. DNA sequence analysis revealed five amino acid differences between human and rhesus. One difference was in the region N-terminal to the I-domain (Thr59Ser) and the other four differences were in the I-domain itself. Va1133Ile, Argl89Gln. Lys197Glu, and Va1308Ala (Fig. 2).

### (d) FACScan analysis of F(ab) and IgG binding to Jurkat cells

Aliquots of 10⁶ Jurkat T-cells were incubated with serial dilutions of humanized and control antibodies in PBS-0.1% BSA-10 mM sodium azide for 45 min at 4°C. The cells were washed and then incubated in fluorescein-conjugated goat anti-human F(ab')₂ (Organon Teknika, Westchester, PA) for 45 min at 4°C. Cells were washed and analysed on a FACScan (Becton Dickinson, Mountain View, CA). 8 x 10³ cells were acquired by list mode and gated by forward light scatter versus side light scatter, thereby excluding dead cells and debris.

### (e) Saturation binding to determine apparent K_{d}s

Radiolabeled antibodies were prepared using lodo-Gen (Pierce, Rockford, IL) according to the manufacturer's instructions. 50 µg of antibody and 1 mCi¹²⁵I (DuPont. Wilmington, DE) were added to each tube and incubated for 15 min at 25° C. Radiolabeled proteins were purified from the remaining free ¹²⁵I using PD-10 columns (Pharmacia, Uppsala, Sweden) equilibrated in Hank's Balanced Salt Solution (HBSS, Life Technologies, Grand Island, NY) containing 0.2% gelatin.

Mononuclear cells were purified from heparinized human peripheral blood collected from two donors using Lymphocyte Separation Medium (LSM, Organon Teknika, Durham, NC) according to the manufacturer's instructions. The blood was centrifuged at 400 x g for 40 min at 25° C with no braking. Cells at the interface of the LSM and plasma were harvested and then resuspended in HBSS-0.2% gelatin.

Leukocytes were purified from heparinized rhesus monkey peripheral blood collected from two individuals by Dextran sedimentation. Blood was diluted with an equal volume of 3% Dextran T500 (Pharmacia) in PBS and was allowed to sediment undisturbed at 25°C for 30 min. After sedimentation, cells remaining in suspension were harvested and pelleted by centrifugation at 400 x g for 5 min. Residual erythrocytes were removed by two cycles of hypotonic lysis using distilled water and 2X HBSS. After erythrocyte lysis, cells were washed in PBS and then resuspended in HBSS-0.2% gelatin.

Antibody affinities were determined by saturation binding using either peripheral blood mononuclear cells (murine MHM24 and HuIgG1) or rhesus leukocytes (MHM23, RhIgG1). In each assay, a radiolabeled antibody was serially diluted in HBSS-0.2% gelatin in quadruplicate. Nonspecific binding was determined by the addition of 500nM final concentration of homologous unlabeled antibody in duplicate through the serial dilution. Human lymphocytes or rhesus leukocytes were added to the plates in a volume of 170 µl per well. Plates were incubated for 2 hr at room temperature on an orbital plate shaker. After incubation, cells were harvested using a SKATRON^{™} cell harvester (Lier, Norway) and washed 10 times with PBS containing 0.25% gelatin and 0.1% sodium azide. Samples were then counted for I min in an LBK Wallac GammaMaster gamma counter (Gaithersburg, MD). Data was transformed from counts per minute to nanomolarity and four-parameter curve-fitting of saturation plots (bound versus total) was then performed to determine K_{d} (app) values.

### (f) Keratinocyte monolayer adhesion assay

Normal human epidermal keratinocytes (Clonetics, San Diego, CA) were removed from culture flasks with trypsin-EDTA, centrifuged, and resuspended in lymphocyte assay medium (RPMI 1640 (GIBCO)-10% fetal calf serum-1% penicillin/streptomycin) at a concentration of 5 x 10⁵ viable cells/ml. Aliquots of 0.1 ml/well were then cultured overnight in flat-bottom 96-well plates; appropriate wells were stimulated by addition of interferon-gamma (Genentech, South San Francisco, CA) at 100 units/well.

Jurkat clone E6-1 cells (ATCC, Rockville, MD) or purified rhesus lymphocytes (see MLR methods) were labeled with 20 µg/ml Calcein AM (Molecular Probes, Eugene, OR) at 37°C for 45 min. After washing three times with lymphocyte assay medium. Jurkat or rhesus lymphocyte cells were resuspended to I x 10⁶ cells/ml and incubated with serially-diluted antibody at 4° C for 30 min. After removal of medium from the keratinocyte monolayer. 0.1 ml/well of labeled cells were added and incubated at 37°C for 1 h. The wells were washed five times with 0.2 ml/well/wash of 37°C lymphocyte medium to remove non-attached cells. Fluorescence was measured using a Cytofluor 2300 (Millipore, Bedford, MA).

A rhesus-human chimeric CD11a (Rh/HuCD1 la) comprising human CD11a with a rhesus 1-domain was constructed by site-directed mutagenesis (Kunkel. *Proc. Natl. Acad Sci. USA* 82:488 (1985)) on a deoxyuridine-containing template plasmid encoding human CD11a. Four residues were altered: Val133Ile, Arg189Gln, Lys197Glu, and Val308Ala (Fig. 2). Plasmids coding for Rh/HuCD11a and human CD11b (EP 364, 690) were cotransfected into an adenovirus-transformed human embryonic kidney cell line, 293 (Graham *et al*., *J*. *Gen. Virol.* 36:59 (1977)), using a high efficiency procedure (Graham *et al*., *J*. *Gen. Virol.* 36:59 (1977); Gorman *et al*., *Science,* 221:551 (1983)). Rh/HuCD11a-transfected 293 cells were labeled with 20 µg/ml Calcein AM at 37°C for 45 min. After washing three times with lymphocyte assay medium, Rh/HuCD11a-transfected 293 cells were resuspended to 1 × 10⁶ cells/ml and incubated with serially-diluted antibody at 4° C for 30 min. After removal of medium from the keratinocyte monolayer, 0.1 ml/well of labeled 293 cells was added and incubated at 37°C for 1 h. The wells were washed five times with 0.2 ml/well/wash of 37°C lymphocyte medium to remove non-attached cells. Fluorescence was measured using a Cytofluor 2300.

### (g) ICAM adhesion assay

Maxisorp (Nunc) 96-well plates were coated with 0.1 ml/well of I µg/ml goat anti-human IgG Fc (Caltag) for I h at 37° C. After washing the plates three times with PBS, the plates were blocked with 1% BSA-PBS for 1 h at 25°C. The plates were then washed three times with PBS and 0.1 ml/well of 50 ng/ml recombinant human ICAM-IgG was added and incubated overnight. The ICAM-IgG consisted of the five extracellular domains of human ICAM fused onto a human IgG Fc. A plasmid for the expression of a human ICAM-1 (Simmons *et al. Nature* 331:624-627 (1988) and Staunton *et al. Cell* 52:925-933 (1988)) immunoadhesin called pRK.5dICAMGalg was constructed. It contains the five Ig-like domains of ICAM-1, a six amino acid cleavage site recognized by an H64A variant of subtilisin BPN', Genenase I (Carter *et al. Proteins: Structure*, *Function and Genetics* 6:240-248 (1989)), and the Fc region from human lgG1 (Ellison *et al*. *Nucleic Acids Research* 10:4071-4079 (1982)) in the pRK5 vector (Eaton *et al*. *Biochemistry* 25:8343-8347 (1986)). Human embryonic kidney 293 cells (Graham *et al*. *J*. *Gen. Virol*. 36:59 (1977)) were stably transfected with pRK.5dICAMGaIg and the RSV-neo plasmid (Gorman *et al*. *Science* 221:551-553 (1983)) to generate a cell line expressing the five domain ICAM 1g (5dICAMlg). A clone was selected which expressed 20 µg/ml of secreted 5dICAMIg by enzyme-linked immunosorbent assay (ELISA), using antibodies to human IgG Fc (Caltag, Burlingame, CA) and ICAM-I (BBIG-II; R & D Systems, Minneapolis, MN). Cell culture supernatant from this cell line was loaded onto a Protein A column (ProsepA, Bioprocessing, Ltd., Durham, England) equilibrated in 0.01 M Hepes buffer (pH 7.0), 0.15 M NaCl (HBS) and the column was washed with HBS followed by 0.01 M Hepes buffer (pH 7.0), 0.5 M NaCl, 0.5 M TMAC (tetra-methyl ammonium chloride) to remove non-specifically bound material. The TMAC buffer was thoroughly washed from the column with HBS and the 5dICAMIg eluted with 0.0 M Hepes buffer (pH 7.0), 3.5 M MgC12 and 10% (w/v) glycerol. The protein A pool was dialyzed extensively against HBS and concentrated.

Purified rhesus lymphocytes (see MLR methods) were labeled with 20 µg/ml Calcein AM (Molecular Probes, Eugene. OR) at 37°C for 45 min. After washing three times with lymphocyte assay medium, rhesus lymphocyte cells were resuspended to 1 x 10⁶ cells/ml and incubated with serially-diluted antibody at 4° C for 30 min. After removal of medium from the ICAM-IgG coated plates. 0.1 ml/well of labeled cells were added and incubated at 37° C for 1 h. The wells were washed five times with 0.2 ml/well/wash of 37° C lymphocyte medium to remove non-attached cells. Fluorescence was measured using a Cytofluor 2300 (Millipore, Bedford, MA).

### (h) One-way mixed lymphocyte response (MLR)

For both human and rhesus MLR, peripheral blood lymphocytes from two unrelated donors were isolated from whole, heparinized blood using Lymphocyte Separation Medium (Organon Teknika, Durham, NC). Lymphocytes were resuspended to a concentration of 3 x 10⁶ cells/ml in RPMI 1640 (GIBCO)-10% human AB serum-1% glutamine-1% penicillin/streptomycin-1% non-essential amino acids-1% pyruvate-5 x 10⁻⁵ M 2-β-mercaptoethanol-50 µg/ml gentamycin-5 µg/ml polymyxin B. The stimulator cells were made unresponsive by irradiation with 3000 rads in a cesium irradiator. Responder cells at a concentration of 1.5 x 10⁵ cells per well were co-cultured with an equal number of stimulator cells in 96-well, flat-bottom plates. Serial two-fold dilutions of each antibody were added to the cultures to give a total volume of 200 µl/well. The cultures were incubated at 37°C in 5% CO2 for 5 days and then pulsed with I µCi/well of [³H]thymidine for 16 h. [³H]thymidine incorporation was measured with a Beckman scintillation counter. Assays were done in triplicate. A humanized anti-human p185^{HER2} MAb (Carter *et al*., *Proc. Natl*. *Acad Sci. USA* 89:4285 (1992)) was used as isotype control for HuIgG1 and RhIgG1. A murine anti-hamster tPA MAb (Genentech) was used as isotype control (murine IgG1) for the MHM23 MAb. MAb 25.3 was purchased from Immunotech, Inc. (Westbrook, ME).

### (i) Computer graphics models of m urine and humanized MHM24

Sequences of the VL and VH domains (Figs. 1A & B) were used to construct a computer graphics model of the murine MHM24 VL-VH domains. This model was used to determine which framework residues should be incorporated into the humanized antibody. A model of F(ab)-8 was also constructed to verify correct selection of murine framework residues. Construction of the models was performed as described previously (Carter *et al*., *Proc. Natl*. *Acad Sci. USA* 89:4285 (1992); Eigenbrot *et al*., *J*. *Mol. Biol*. 229:969 (1993)).

### Results

### (a) Humanization

The consensus sequence for the human heavy chain subgroup III and the light chain subgroup κ I were used as the framework for the humanization (Kabat *et al*., *Sequences of Proteins of Immunological Interest.* 5th Ed. Public Health Service, National Institutes of Health, Bethesda, MD. (1991)) (Figs. 1A & B). This framework has been successfully used in the humanization of other murine antibodies (Carter *et al*., *Proc. Natl*. *Acad*. *Sci. USA* 89:4285 (1992); Presta *et al*., *J*. *Immunol*. 151:2623-2632 (1993); Eigenbrot *et al*., *Proteins* 18:49-62 (1994)). All humanized mutants were initially made and screened for binding as F(ab)s expressed in *E. coli*. Typical yields from 500 ml shake flasks were 0.2-0.5 mg F(ab). Mass spectrometry verified the mass of each F(ab) to within 5 mass units.

CDR-H 1 included residues H28-H3S, which includes all exposed residues from both Kabat *et al*., *Sequences of Proteins of Immunological Interest*. 5th Ed. Public Health Service, National Institutes of Health, Bethesda, MD. (1991) and Chothia *et al*. *Nature* 342:8767 (1989). The other hypervariable loops were defined according to Chothia *et al.* (1989). Light chain residue numbers are prefixed with L: heavy chain residue numbers are prefixed with H.

**Table II**

| *Binding of humanized MHM24 mutants to human CD11a on Jurkat cells* | | | | | | |
|---|---|---|---|---|---|---|
| | | | | EC50 F(ab)/ | | |
| | | | | EC50 F(ab)-2^{b} | | |
| Mutant | Template | Changes^{a} | Purpose | Mean | S.D. | N |
| F(ab)-1 | Human FR | ArgH71Val | Straight CDR swap | no binding | | 2 |
| F(ab)-2 | F(ab)-1 | AlaH60Asn | Extended CDR-H2 | 1.0 | | 4 |
| | | AspH61 Gln | (Kabat *et al.* (1991)) | | | |
| | | SerH62Lys | | | | |
| | | ValH63Phe | | | | |
| | | GlyH65Asp | | | | |
| F(ab)-3 | F(ab)-2 | PheH67Ala | Packing;CDR-H2 | 1.2 | 0.33 | 3 |
| F(ab)-4 | F(ab)-2 | ValH71Arg | Packing;CDR-H1,H2 | 2.9 | | 1 |
| F(ab)-5 | F(ab)-2 | AsnH73Lys | Framework loop in VH | 0.043 | 0.015 | 4 |
| | | LysH75Ser | | | | |
| | | AsnH76Ser | | | | |
| F(ab)-6 | F(ab)-5 | SerL53Thr | Extended CDR-L2 | 0.012 | 0.005 | 4 |
| | | GluL55Gln | | | | |
| F(ab)-7 | F(ab)-6 | PheH27Tyr | Extended CDR-H1 | 0.004 | 0.002 | 4 |
| F(ab)-8 | F(ab)-7 | SerH75Lys | Framework loop in VH | 0.004 | 0.002 | 4 |
| | | SerH76Asn | back to human | | | |
| HulgG1^{c} | | | | 0.004 | 0.002 | 4 |
| chlgG1^{d} | | | | 0.006 | 0.005 | 4 |

| | | | | | | |
|---|---|---|---|---|---|---|
| ^{a} Murine residues are in bold; residue numbers are according to Kabat *et al*., *Sequences of Proteins of Immunological Interest* 5th Ed. Public Health Service, National Institutes of Health, Bethesda, MD (1991). ^{b} Mean and standard deviation are the average of the ratios calculated for each of the independent FACScan assays; the EC50 for F(ab)-2 was 771 ± 320 ng/ml. ^{c} HuIgG1 is F(ab)-8 VL and VH domains fused to human constant light and heavy chains. ^{d} chIgG1 is chimeric IgG with murine VL and VH domains fused to human constant light and heavy chains. | | | | | | |

In the initial mutant F(ab)- the CDR residues were transferred from the murine antibody to the human framework. In addition, residue H71 was changed from the human Arg to murine Val since this residue has been shown previously to affect the conformations of CDR-H1 and CDR-H2 (Chothia *et al*., *Nature,* 342:8767-883 (1989); Tramantano *J*. *Mol. Biol.* 215:175 (1990)). This F(ab) showed no detectable binding. In F(ab)-2 CDR-H2 was extended to include the sequence-based definition (*i*.*e*., including residues H60-H65). The EC50 for F(ab)-2 binding to human CD11a was 771 ± 320 ng/ml, which was 148-fold weaker than the EC50 of the chimeric IgG1 (5.2 ± 3.0 ng/ml).

In previous humanizations, it has been found that residues in a framework loop (FR-3) adjacent to CDR-H1 and CDR-H2 can affect binding (Eigenbrot *et al*., *Proteins* 18:49-62 (1994)). In F(ab)-5 three residues in this loop were changed to their murine counterpart and this mutant showed a 23-fold improvement in binding (Table II). Alteration of human residues at positions L53 and L55 to murine (*i*.*e*. SerL53Thr and GluL55Gln) further improved binding by another4-fold (F(ab)-6. Table II): this effectively converted CDR-L2 from the structure-based definition (residues L50-L52) to the sequence-based definition (residues L50-L56). Subsequent alteration of PheH27 to murine Tyr in CDR-H1 resulted in an additional 3-fold improvement (F(ab)-7; Table II). Finally, based on the models of murine and humanized MHM24, two of the three murine residues (H75 and H76) in FR-3 were changed back to human and it was found that these two residues had no effect on binding (cf. F(ab)-7 and F(ab)-8, Table II). The average EC50 for F(ab)-8 was slightly better than that of the chimeric IgG1 (Table II). Not all changes from human to murine resulted in improved binding. PheH67 was changed to murine Ala since this position had been previously found to affect binding (Presta *et al*., *J*. *Immunol.* 151:2623-2632 (1993)) but no effect was evident (F(ab)-3. Table II). Changing ValH71 back to the human Arg effected a 3-fold reduction in binding (F(ab)-4, Table II), supporting inclusion of ValH71 in F(ab)-1.

The VL and VH domains from F(ab)-8 were transferred to human IgG I constant domains. The full length intact antibody, HulgG1, showed an EC50 equivalent to F(ab)-8 and improved compared to the full length chimeric IgG1 (Table II). When data for all assays of HulgG1 is considered, including its use as a standard for the alanine-scan and MLR assays (see below), the EC50 for HulgG1 against human CD11a was 0.042 ± 0.072 nM (N = 15). Saturation binding analysis was also performed to determine the apparent dissociation constants, K_{d}(app): 0.15 ± 0.02 nM for murine MHM24 and 0.15 ± 0.04 nM for HulgG I (Table III).

**Table III**

| *Apparent K_{d} by saturation binding to human lymphocytes and rhesus leukocytes* | | | | |
|---|---|---|---|---|
| | muMHM24 | HuIgG1 K_{d}(app) | muMHM23 K_{d}(app) | RhIgG1 K_{d}(app) |
| | K_{d}(app) | nM | nM | nM |
| | nM | | | |
| human donor 1 | 0.16 +/- 0.01 | 0.11 +/- 0.08 | | |
| human donor 2 | 0.13 +/- 0.02 | 0.18 +/- 0.03 | | |
| rhesus donor 1 | | | 3,9 +/- 0.31 | 3.9 +/- 1.04 |
| rhesus donor 2 | | | 4.5 +/- 0.51 | n.d. |
| rhesus donor 3 | | | | 2.8 +/- 1.1^{a} |
| rhesus donor 3 | | | | 2.7+/-0.9 |

| | | | | |
|---|---|---|---|---|
| ^{a} Assays of rhesus donor 3 were performed using two batches of RhIgG1; the assays were performed in the presence of 1 mg/ml human IgG1 to block Fc receptor interaction. | | | | |

### (b) Alanine-Scan of CDR Residues

To determine which CDR residues were involved in binding to human CD11a an alanine-scan (Cunningham *et al*., *Science* 244:1081 (1989)) was performed on the CDR residues of HuIgG1. Each mutant was tested for binding to CD11a on Jurkat cells. In the light chain only CDR-L3 contributes to binding. HisL91 had a large effect (Table IV) and is probably conformational since this side chain should be partially buried. Residues AsnL92 and TyrL94 had more modest effect, reducing binding by 3-fold and 12-fold, respectively. Note however that simultaneously changing these two residues to alanine (as well as GluL93Ala) had a non-additive effect on binding (mutant L3, Table IV).

**Table IV**

| *Alanine-scan of humanized MHM24 CDR residues* | | | | | | | | | | | | | | | | | | | | | | | | |
|---|---|---|---|---|---|---|---|---|---|---|---|---|---|---|---|---|---|---|---|---|---|---|---|---|
| **Mutant IgG1** | | | | | | | | | | | | | | | | | | | **Human CD11a** | | | **Rhesus CD11a** | | |
| | | | | | | | | | | | | | | | | | | | **Var.EC50/HulG1** | | | **Var.EC50/HulgG1** | | |
| | | | | | | | | | | | | | | | | | | | **EC50^{b}** | | | **EC50^{c}** | | |
| **CDR Sequence^{a}** | | | | | | | | | | | | | | | | | | | **Mean** | **S.D.** | **N** | **Mean** | **S.D.** | **N** |
| CDR-H1 | G | Y | S | F | T | G | H | W | M | N | | | | | | | | | | | | | | |
| H1^{d} | | | A | | A | | A | | | | | | | | | | | | 5.9 | 0.8 | 2 | nb | | |
| SerH28Ala | | | A | | | | | | | | | | | | | | | | 6.9 | 0.1 | 2 | nb | | |
| ThrH30Ala | | | | | A | | | | | | | | | | | | | | 1.7 | 0.3 | 2 | 1.3 | | |
| GlyH31Ala | | | | | | A | | | | | | | | | | | | | 1.2 | 0.1 | 2 | 2.4 | | |
| HisH32Ala | | | | | | | A | | | | | | | | | | | | 2.3 | 0.2 | 2 | nb | | |
| TrpH33Ala | | | | | | | | A | | | | | | | | | | | >870 | | 1 | nb | | |
| | | | | | | | | | | | | | | | | | | | | | | | | |
| CDR-H2 | | M | I | H | P | S | D | S | E | T | R | Y | N | Q | K | F | K | D | | | | | | |
| H2 | | | | A | | A | | A | | | | | | | | | | | 14.1 | 7.8 | 10 | 0.055 | 0.050 | 15 |
| H2B | | | | | | | A | | A | | A | | | | | | | | >600 | | 2 | nb | | |
| H2A1 | | | | A | | A | | | | | | | | | | | | | 10.8 | 7.3 | 6 | 0.013 | 0.012 | 10 |
| H2A2 | | | | | | A | | A | | | | | | | | | | | 1.9 | 0.1 | 2 | 1.1 | 0.1 | 2 |
| H2A3 | | | | A | | | | A | | | | | | | | | | | 4.6 | 0.2 | 2 | 1.3 | | |
| HisH52Ala | | | | A | | | | | | | | | | | | | | | 1.5 | 0.2 | 2 | 0.7 | | |
| HisH52Ser | | | | S | | | | | | | | | | | | | | | 5.0 | 0.3 | 2 | nb | | |
| SerH53Ala | | | | | | A | | | | | | | | | | | | | 1.8 | 0.1 | 2 | 0.7 | | |
| AspH54Ala | | | | | | | A | | | | | | | | | | | | 147 | 18 | 2 | 0.3 | | |
| SerH55Ala | | | | | | | | A | | | | | | | | | | | 1.3 | 0.1 | 2 | 1.3 | | |
| SerH55Asn | | | | | | | | N | | | | | | | | | | | 2.1 | 0.2 | 2 | nb | | |
| SerH55Gln | | | | | | | | Q | | | | | | | | | | | 2.9 | 1.4 | 2 | 6.7 | | |
| GluH56Ala | | | | | | | | | A | | | | | | | | | | 4.0 | 0.6 | 2 | 0.3 | | |
| ArgH58Ala | | | | | | | | | | | A | | | | | | | | 1.1 | 0.1 | 2 | 3.3 | | |
| GlnH61Ala | | | | | | | | | | | | | | A | | | | | 4.1 | 0.1 | 2 | 5.3 | | |
| LysH62Ala | | | | | | | | | | | | | | | A | | | | 1.8 | 0.2 | 2 | 4.9 | | |
| LysH64Ala | | | | | | | | | | | | | | | | | A | | 2.5 | 0.1 | 2 | 1.2 | | |
| AspH65Ala | | | | | | | | | | | | | | | | | | A | 0.8 | 0.1 | 2 | 1.1 | | |
| | | | | | | | | | | | | | | | | | | | | | | | | |
| FR-3 | | | | | | | | | | | | | | | | | | | | | | | | |
| LysH73Ala | | | | | | | | | | | | | | | | | | | 5.2 | 0.9 | 2 | nb | | |
| LysH73Arg | | | | | | | | | | | | | | | | | | | 4.6 | 1.1 | 2 | 5.5 | | |
| | | | | | | | | | | | | | | | | | | | | | | | | |
| CDR-H3 | G | I | Y | F | Y | G | T | T | Y | F | D | | | | | | | | | | | | | |
| H3 | | | A | | A | | | | | | | | | | | | | | >900 | | 2 | nb | | |
| H3B | | | | | | | | A | A | A | | | | | | | | | 34.7 | 13.6 | 2 | nb | | |
| TyrH97Ala | | | A | | | | | | | | | | | | | | | | 10.9 | 2.1 | 2 | nb | | |
| TyrH99Ala | | | | | A | | | | | | | | | | | | | | 1.4 | 0.1 | 2 | nb | | |
| TbrH100aAla | | | | | | | A | | | | | | | | | | | | 2.3 | 0.6 | 2 | nb | | |
| ThrH100bAla | | | | | | | | A | | | | | | | | | | | 1.4 | 0.2 | 2 | nb | | |
| TyrH100cAla | | | | | | | | | A | | | | | | | | | | 7.6 | 1.0 | 2 | nb | | |
| | | | | | | | | | | | | | | | | | | | | | | | | |
| CDR-L1 | R | A | S | K | T | I | S | K | Y | L | A | | | | | | | | | | | | | |
| L1 | | | | A | A | | A | A | | | | | | | | | | | 1.3 | 0.3 | 3 | 1.0 | | |
| | | | | | | | | | | | | | | | | | | | | | | | | |
| CDR-L2 | S | G | S | T | L | Q | S | | | | | | | | | | | | | | | | | |
| L2 | A | | A | A | | | | | | | | | | | | | | | 1.1 | 0.0 | 2 | nb | | |
| SerL50Ala | A | | | | | | | | | | | | | | | | | | 1.2 | 0.5 | 2 | 2.7 | | |
| SerL52Ala | | | A | A | | | | | | | | | | | | | | | 1.1 | 0.2 | 2 | 13.3 | | |
| ThrL53Ala | | | | A | | | | | | | | | | | | | | | 0.9 | 0.4 | 2 | 0.7 | | |
| | | | | | | | | | | | | | | | | | | | | | | | | |
| CDR-L3 | Q | Q | H | N | E | Y | P | L | T | | | | | | | | | | | | | | | |
| L3 | | | | A | A | A | | | | | | | | | | | | | >900 | | 2 | nb | | |
| HisL91Ala | | | A | | | | | | | | | | | | | | | | >900 | | 2 | nb | | |
| AsnL92Ala | | | | A | | | | | | | | | | | | | | | 3.3 | 0.7 | 2 | 3.3 | | |
| GluL93Ala | | | | | A | | | | | | | | | | | | | | 1.7 | 0.2 | 2 | 2.9 | | |
| TyrL94Ala | | | | | | A | | | | | | | | | | | | | 11.8 | 0.1 | 2 | nb | | |
| | | | | | | | | | | | | | | | | | | | | | | | | |
| hu4D5^{e} | | | | | | | | | | | | | | | | | | | >900 | | 5 | nb | | |

| | | | | | | | | | | | | | | | | | | | | | | | | |
|---|---|---|---|---|---|---|---|---|---|---|---|---|---|---|---|---|---|---|---|---|---|---|---|---|
| ^{a} CDRs and FR-3 are as defined in Kabat *et al.,* (1991) *supra.* ^{b} EC50 HuIgG1 for human CD11 a = 0.042 nM (S.D.= 0.072; N = 15). ^{c} EC50 HulgG for rhesusCD11a = 45.6 nM (S.D.= 40.4; N = 16); all values for rhesus CD11a are for a single binding assay unless otherwise noted; nb denotes binding of mutant is greater than 10-fold weaker than HuIgG1. ^{d} Multiple alanine mutants: H1, SerH28Ala/ThrH30Ala/HisH32Ala; H2,HisH52Ala/SerH53Ala/SerH55Ala; H2B, AspH54Ala/GluH56Ala/ArgH58Ala; H2A1, HisH52Ala/SerH53Ala; H2A2, SerH53Ala/SerH55Ala; H2A3, HisH52Ala/SerH55Ala; H3,TyrH97Ala/TyrH99Ala; H3B, ThrH 100aAla/ThrH100bAla/TyrH 100Ala; L1, LysL27Ala/ThrL28Ala/SerL30Ala/LysL31Ala; L2, SerL50Ala/SerL52Ala/ThrL53Ala; L3 AsnL92Ala/GluL93Ala/TyrL94Ala. ^{e} hu4D5 is a humanized anti-p 185^{HER2} antibody with the same IgG1 framework as the huMHM24 antibody (Carter *et al*., *Proc*.*Natl*. *Acad*. *Sci. USA* 89:4285 (1992)). | | | | | | | | | | | | | | | | | | | | | | | | |

In the heavy chain, CDR-H2 and CDR-H3 are the prominent contributors to the binding. CDR-H1 residue TrpH33Ala had a large effect but this is most likely due to a conformational change as TrpH33 should be partially buried. The most important single residue contributing to the binding is AspH54 in CDR-H2; changing this residue to alanine effected a 147-fold reduction in binding (Table IV). Other residues in CDR-H2 involved in binding include GluH56. GlnH61 and LysH64 (Table IV). In CDR-H3, TyrH97Ala reduced binding by 11-fold and TyrH100cAla by 8-fold. As in CDR-L3. simultaneous alteration of several CDR-H3 residues to alanine effected a non-additive, large reduction in binding (cf. mutant H3 versus TyrH97Ala and TyrH99Ala, Table IV). In addition, the FR-3 residue included in the humanization, LysH73, also showed a 5-fold reduction in binding when changed to alanine or arginine (Table IV).

### (c) Re-engineering HulgG1 to Bind to Rhesus CD11a

Both murine MHM24 and HulgG1 showed approximately 1000-fold reduction in binding to rhesus CD11a: HulgG1 had an EC50 against rhesus CD11a of 45.6 ± 40.4 nM (N = 16) compared to an EC50 of 0.042 ± 0.072 nM against human CD11a. Since a primate model is important in evaluating the biology, toxicity, and efficacy of MHM24. improving the binding of HulgG1 to rhesus CD11a was deemed advantageous. Initially, the MAb hypervariable region residues which were important in binding to human CD11a and rhesus CD11a were determined so that those important for the rhesus but not the human could be altered. Accordingly, the alanine-scan mutants were also assayed against rhesus CD11a on peripheral blood lymphocytes. The most important finding was that one of the multiple-alanine mutation mutants, mutant H2, bound 18-fold better to rhesus CD11a than HuIgG1 (Table IV). However, individual mutations at the three residues included in mutant H2 showed minimal improvement in binding: HisH52Ala, 0.7-fold better, SerH53Ala, 0.7-fold better, and SerH55Ala, 1.3-fold worse (Table IV). A series of double mutations at these three residues showed that the combination HisH52Ala-SerH53Ala was the best, providing a 77-fold improvement in binding compared to HuIgG1 (cf. mutants H2A1, H2A2 and H2A3, Table IV). In addition, the AspH54Ala and GluH56Ala mutants also effected a 3-fold improvement over HuIgG1 (Table IV), even though AspH54 is the most important binding residue in HuIgG1 with respect to human CD11a.

In an attempt to find a single substitution at position H54 which would improve binding to rhesus CD11a but not reduce binding to human CD11a, position H54 was substituted with a variety of amino acids. All substitutions reduced binding by greater than an order of magnitude whereas the substitution AspH54Asn improved rhesus binding by 10-fold (Table V).

**Table V**

| *Amino acid substitution at AspH54* | | | |
|---|---|---|---|
| AspH54 change to | Mutant EC50/HulgG1 EC50^{a} | | |
| | Human CD11a^{b} | | Rhesus CD11a^{c} |
| | mean | S.D. | |
| Ala | 147 | 18 | 0.3 |
| Asn | 26 | 1 | 0.1 |
| Gln | 20 | 1 | 4.4 |
| Glu | 16 | 2 | >25 |
| Ser | >100 | | 0.9 |
| Arg | >250 | | >25 |
| Lys | >100 | | 3.8 |
| His | >300 | | >25 |
| Thr | >450 | | >25 |
| Met | > 150 | | >25 |
| Leu | >300 | | >25 |

| | | | |
|---|---|---|---|
| ^{a} EC50 HulgG1 for human CD11a = 0.042 nM (S.D.= 0.072; N=15); EC50 HulgG1 for rhesus CD11a = 45.6 nM (S.D.= 40.4; N=16). ^{b} Values are the mean of two assays. ^{c} Values are for a single assay. | | | |

Since non-additive effects were noted for changes at positions H52-H53, these were combined with a variety of changes at positions H54 and H56 (Table VI). For all mutants, H52 and H53 were alanine. In one series, position H54 was Asn and position H56 was Glu (original), Ala, Asn or Gln. None of these mutants improved rhesus CD11a binding over the H2A 1 mutant (Table VI). In another series, position H54 was Ala and position H56 was Glu (original), Ala, Ser or Asn and again all were worse than mutant H2A1. In the third series, position H54 was Ser and position H56 was Glu (original), Ala, Ser or Asn. Two of these mutants exhibited improved binding to rhesus CD11a compared to the H2A1 mutant (H2C11 and H2C12, Table VI). The rhesus CD 11a EC50 for these two mutants was 0.11 ± 0.11 nM (N = 9) for H2C11 and 0.19 ± 0.08 nM (N = 7) for H2C12. These values are 3- to 5-fold weaker than the EC50 of HulgG1 for human CD11a (0.042 nM) but are a 240- to 415-fold improvement over the EC50 of HulgG1 for rhesus CD 11a (45.6 nM). H2C 12 will hereafter be referred to as RhIgG1. Apparent K_{d} values from saturation binding experiments showed that RhlgG1 bound to rhesus CD11a as well as murine MHM23 bound to rhesus CD18 (Table III).

**Table VI**

| *Binding of CDR-H2 mutants to human and rhesus CD11a* | | | | | | | | | | | | | | |
|---|---|---|---|---|---|---|---|---|---|---|---|---|---|---|
| **Mutant IgG1** | **Sequence** | | | | | | | | | | | **Var.EC50/HulgG1 EC50^{a}** | | |
| | | | | | | | | | | | | **Human CD11a** | **Rhesus CD11a** | |
| | | | | | | | | | | | | | **Mean** | **S.D.** |
| H2C2 | | | | A | | | | | | | | 2.6 | >100 | |
| H2C3 | | | A | A | A | N | | | | | | >100 | >100 | |
| CDR-H2 | M | I | H | P | S | D | S | E | T | R | Y | 1.0 | 1.00 | |
| H2A1 | | | A | | A | | | | | | | 10.8 | 0.013 | 0.012 (N=10) |
| H2C1 | | | A | | A | N | | | | | | >100 | 0.56 | 0.01 |
| H2C4 | | | A | | A | N | | A | | | | >100 | 0.38 | 0.06 |
| H2C5 | | | A | | A | N | | N | | | | 46 | 0.11 | 0.02 |
| H2C6 | | | A | | A | N | | Q | | | | > 100 | 0.21 | 0.01 |
| H2C8 | | | A | | A | A | | | | | | 12.7 | 0.38 | |
| H2C7 | | | A | | A | A | | A | | | | 2.4 | 1.03 | 0.05 |
| H2C10 | | | A | | A | A | | S | | | | 14.2 | 0.22 | 0.03 |
| H2C9 | | | A | | A | A | | N | | | | 34.3 | 0.22 | 0.04 |
| H2C13 | | | A | | A | S | | | | | | | 0.10 | 0.06 |
| H2C14 | | | A | | A | S | | A | | | | | 0.021 | 0.013 |
| H2C12 | | | A | | A | S | | S | | | | | 0.004 | 0.001 (N=7) |
| H2C11 | | | A | | A | S | | N | | | | 24.9 | 0.002 | 0.001 (N=9) |

| | | | | | | | | | | | | | | |
|---|---|---|---|---|---|---|---|---|---|---|---|---|---|---|
| ^{a} EC50 HuIgG1 for human CD11 a = 0.042 nM (S.D.= 0.072; N = 15); EC50 HuIgG1 for rhesus CD11a = 45.6 nM (S.D.= 40.4; N = 16); all values for rhesus CD11 a are the mean of two independent binding assays except as noted. | | | | | | | | | | | | | | |

For the HuIgG1-human CD11a interaction, AspH54 was the most important residue (Table IV); changing this residue to other amino acids significantly reduced binding with the least reduction occurring for changes to Glu, Asn and Gln. However, for the HulgG1-rhesus CD11a interaction, AspH54 was deleterious since changing this residue to Ala or Asn improved binding (Table V). In order to understand this difference between binding to human and rhesus CD11a, the latter was cloned from a rhesus PBL library. Fig. 2 shows that rhesus CD11a I-domain differs from human CD11a I-domain at only four positions: 133, 189, 197, 308. Previously the human CD11a epitope of MHM24 was mapped to residues 197-203 (Champe *et al., J. Biol. Chem.* 270:1388-1394 (1995)) which includes the human Lys197 to rhesus Glu197 change in rhesus.

### (d) Keratinocyte Cell Adhesion Assays

Murine MHM24, chimeric IgG1 and HuIgG1 were compared in their ability to prevent adhesion of Jurkat cells (human T-cells expressing LFA-1) to normal human epidermal keratinocytes expressing ICAM-1. All three antibodies performed similarly (Fig. 3) with similar IC50 values (Table VII).

**Table VII**

| *Blocking of cell adhesion by MHM24 mutants* | | | | | | | | | | |
|---|---|---|---|---|---|---|---|---|---|---|
| | IC50 Value (nM) | | | | | | | | | |
| mAb | Jurkat:HuK^{a} | RhLy^{b}:HuK | | | RhLy:HuICAM^{c} | | | Rh/HuCD11a^{d}:HuK | | |
| | | Mean | S.D. | N | Mean | S.D. | N | Mean | S.D. | N |
| murMHM24 | 0.09 | | | | | | | | | |
| HuIgG1 | 0.13 | | | | | | | | | |
| chIgG1 | 0.10 | | | | | | | | | |
| RhIgG1 | | 119 | 86 | 4 | 5.3 | 4.5 | 3 | 4.9 | 0.2 | 2 |
| MHM23 | | 1.6 | 1.5 | 3 | 1.2 | | | 1.4 | 0.1 | 2 |

| | | | | | | | | | | |
|---|---|---|---|---|---|---|---|---|---|---|
| ^{a} HuK = normal human epidermal keratinocyte. ^{b} RhLy = rhesus lymphocyte. ^{c} HulCAM = recombinant human ICAM-1. ^{d} Rh/HuCD11a = human CD11a with rhesus I-domain transfected into human 293 cells. | | | | | | | | | | |

Neither murine nor humanized MHM24 blocked rhesus or cynomolgus lymphocytes from adhering to human keratinocytes. When RhIgG1 was compared to the murine anti-human CD18 antibody MHM23 (Hildreth *et al., Eur. J. Immunol.* 13:202-208 (1983); Hildreth *et al., J. Immunol.* 134:3272-3280 (1985)) in blocking adherence of rhesus lymphocytes to human keratinocytes, RhIgG1 was 74-fold less efficacious than MHM23 (Fig. 4A, Table VII). However, when recombinant human ICAM-1 was coated on plates (instead of human keratinocytes) RhIgG1 was only 4-fold less efficacious than MHM23 (Fig. 4B. Table VII). A chimeric CD11a comprised of human CD11a in which the I-domain was mutated to rhesus (Val133Ile, Arg 189Gln, Lys 197Glu, Val308Ala) was transfected into human embryonic kidney 293 cells. Again, RhIgG1 was only 4-fold down from MHM23 in blocking these Rh/HuCD11a-293 cells from adhering to human keratinocytes (Fig. 4C, Table VII).

Control isotype antibodies for RhIgG1 (humanized anti-p 185HER2 antibody; Carter *et al., Proc. Natl. Acad. Sci. USA* 89:4285 (1992)) and MHM23 (murine MAb 354, a murine IgG1 anti-hamster tPA) did not block binding of rhesus lymphocytes to human keratinocytes or recombinant ICAM-1 (Figs. 4A, 4B) or Rh/HuCD11a to human keratinocytes (Fig. 4C). This implies that the reduced performance of RhlgG1 compared to murine MHM23 in the rhesus lymphocyte:human keratinocyte assay was not due to any unexpected interaction of the human Fc of HulgG1 (compared to the murine Fc of MHM23) with the rhesus lymphocytes, which might reduce the concentration of RhIgG1 available for binding to rhesus CD11a. The recombinant human ICAM-1 data show that RhIgG1 is binding to the rhesus lymphocytes and preventing adherence almost as well as murine MHM23 (Fig. 4B, Table VII). The Rh/HuCD11a-293 data (Fig. 4C, Table VII) show that RhIgG1 is not binding to targets on the human keratinocytes (compared to HulgG1), which might reduce the concentration of RhlgG1 available for binding to rhesus CD11a. In addition, the K_{d}(app) of RhIgG1 to rhesus leukocytes was similar with (rhesus donor 3) and without (rhesus donor 1) addition of I mg/ml human IgG1 (Table III); This shows that binding of RhIgG I is specific to rhesus CD11a.

### (e) Mixed Lymphocyte Response Assays

In the MLR, HuIgG1 exhibited an IC50 value 2-fold weaker than the murine MHM24 (Table VIII, Fig. 5).

**Table VIII**

| *Mixed lymphocyte response assay results* | | | |
|---|---|---|---|
| | IC50 Value (nM) | | |
| mAb^{a} | Mean | S.D. | N |
| murMHM24 | 0.19 | 0.06 | 3 |
| HulgG1 | 0.38 | 0.14 | 4 |
| mAb 25.3 | 3.8 | 1.0 | 2 |
| RhlgG1 | 23.4 | 11.4 | 2 |
| MHM23 | 30.4 | 24.0 | 3 |

| | | | |
|---|---|---|---|
| ^{a} murMHM24, HuIgG1 and mAb 25.3 tested in human MLR; RhIgG1 and MHM23 tested in rhesus MLR. | | | |

Both the murine and humanized MAbs fared 10- to 20-fold better than MAb 25.3, which has been previously tested *in vivo* (Fischer *et al*., *Blood* 77:249-256 (1991); Stoppa *et al*., *Transplant Intl*. 4:3-7 (1991); Hourmant *et al*., *Transplantation* 58:377-380 (1994)). The rhesus-binding mutant RhIgG1 exhibited an IC50 value slightly better than murine MHM23 (Table VIII). Different responder:stimulator blood donors were used in independent assays and the results did not vary significantly. The K_{d} of RhIgG1 for rhesus CD11a is about 26-fold down from the K_{d} of HuIgG1 for human CD11a (Table III) and this is reflected in the IC50 values derived from the MLR assays (Table VIII).

## Claims

1. An antibody mutant of a humanized MHM24 anti-human CD11a antibody, which antibody mutant comprises a heavy chain variable domain comprising the amino acid sequence in Seq ID N0:17 and has a binding affinity for CD11a from a nonhuman mammal which is at least about 10 fold stronger than the binding affinity of the humanized MHM24 antibody for said CD11a.

2. The antibody mutant of claim 1 wherein the nonhuman mammal is a primate.

3. The antibody mutant of claim 2 wherein the nonhuman primate is selected from the group consisting of rhesus, cynomolgus, baboon, chimpanzee and macaque.

4. The antibody mutant of claim 1 wherein the antibody mutant has a binding affinity for said CD11 a from the nonhuman mammal which is at least about 20 fold stronger than the binding affinity of the humanized MHM24 anti-human CD11a antibody for said CD11a.

5. The antibody mutant of claim 1 wherein the antibody mutant has a binding affinity for said CD11 a from the nonhuman mammal which is at least about 50 fold stronger than the binding affinity of the humanized MHM24 anti-human CD11a antibody for said CD11a.

6. The antibody mutant of claim 1 further comprising an amino acid substitution in a framework region of said humanized MHM24 anti-human CD11a antibody.

7. A non-therapeutic method of using the mutant antibody of claim 1 in preclinical studies by administering it to a nonhuman mammal.

8. A method for making an antibody mutant, comprising the steps of:
(a) identifying hypervariable region residues in a species-dependent antibody which are involved in binding an antigen from a first mammalian species and those hypervariable region residues involved in binding a homologue of the antigen from a second different mammalian species;
(b) preparing a mutant of the species-dependent antibody wherein a residue identified in (a) as being Involved in binding the antigen from the first mammalian species or the homologue thereof, or both, is replaced by another amino acid residue; and
(c) selecting an antibody mutant prepared as in (b) which has a stronger binding affinity for the antigen from the second mammalian species than the species-dependent antibody.

9. The method of claim wherein the first mammalian species is a human.

10. The method of claim 8 wherein the second mammalian species is a nonhuman mammal.

11. The method of claim 10 wherein the nonhuman mammal is a primate.

12. The method of claim 11 wherein the nonhuman primate is selected from the group consisting of rhesus, cynomolgus, baboon, chimpanzee and macaque.

13. The method of claim 8 wherein the species-dependent antibody is humanized MHM24 anti-human CD11a and the antigen is human CD11a.

14. The method of claim 13 wherein the second mammalian species is rhesus.

15. The method of claim 8 wherein step (b) involves preparing a mutant of the species-dependent antibody wherein a residue identified In (a) as being involved in binding the homologue, but not the antigen from the first mammalian species, is replaced by another amino acid residue.

16. The method of claim 8 wherein step (b) involves preparing a mutant of the species-dependent antibody wherein a residue identified in (a) as being involved in binding both the antigen from the first mammalian species and the homologue thereof is replaced by another amino add residue.

17. The method of claim 8 wherein step (b) involves preparing a mutant of the species-dependent antibody wherein a residue identified in (a) as being involved in binding the antigen from the first mammalian species, but not the homologue thereof, is replaced by another amino acid residue.

18. A heavy chain variable domain comprising the amino acid sequence in SEQ ID NO: 17.

19. isolated nucleic acid encoding the antibody mutant of claim 1.

20. A vector comprising the nucleic acid of claim 19.

21. A host cell transformed with the vector of claim 20.

22. A process of producing an antibody mutant comprising culturing the host cell of claim 21 so that the nucleic acid is expressed.

23. The process of claim 22 further comprising recovering the antibody mutant from the host cell culture.

24. The process of claim wherein 23 the antibody mutant is recovered from the host cell culture medium.

## Patentansprüche

1. Antikörpermutante eines humanisierten MHM24-Anti-Human-CD11a-Antikörpers, wobei die Antikörpermutante eine variable Schwerkettendomäne umfasst, welche die Aminosäuresequenz der Seq.-ID Nr. 17 umfasst, und eine Bindungsaffinität für CD11a aus einem nichtmenschlichen Säugetier aufweist, die zumindest 10-mal höher ist als die Bindungsaffinität des humanisierten MHM24-Antikörpers für das CD11a.

2. Antikörpermutante nach Anspruch 1, worin das nichtmenschliche Säugetier ein Primat ist.

3. Antikörpermutante nach Anspruch 2, worin der nichtmenschliche Primat aus der aus Rhesusaffen, Javaneraffen, Pavianen, Schimpansen und Makaken bestehenden Gruppe ausgewählt ist.

4. Antikörpermutante nach Anspruch 1, worin die Antikörpermutante eine Bindungsaffinität für das CD11a aus dem nichtmenschlichen Säugetier aufweist, die zumindest etwa 20-mal höher ist als die Bindungsaffinität des humanisierten MHM24-Anti-Human-CD11 a-Antikörpers für das CD11a.

5. Antikörpermutante nach Anspruch 1, worin die Antikörpermutante eine Bindungsaffinität für das CD11a aus dem nichtmenschlichen Säugetier aufweist, die zumindest etwa 50-mal höher ist als die Bindungsaffinität des humanisierten MHM24-Anti-Human-CD11 a-Antikörpers für das CD11a.

6. Antikörpermutante nach Anspruch 1, die weiters eine Aminosäuresubstitution in einer Gerüstregion des humanisierten MHM24-Anti-Human-CD11 a-Antikörpers aufweist.

7. Nichttherapeutisches Verfahren zur Verwendung einer Antikörpermutante nach Anspruch 1 in vorklinischen Studien durch Verabreichung derselben an ein nichtmenschliches Säugetier.

8. Verfahren zur Herstellung einer Antikörpermutante, folgende Schritte umfassend:
(a) das Identifizieren von Resten einer hypervariablen Region in einem speziesabhängigen Antikörper, die an der Bindung eines Antigens aus einer ersten Säugetierspezies beteiligt sind, und von jenen Resten einer hypervariablen Region, die an der Bindung eines Homologs des Antigens aus einer zweiten, unterschiedlichen Säugetierspezies beteiligt sind;
(b) das Herstellen einer Mutante des speziesabhängigen Antikörpers, worin ein Rest, der unter (a) als an der Bindung des Antigens aus der ersten Säugetierspezies oder des Homologs davon oder an beiden beteiligt identifiziert wurde, durch einen anderen Aminosäurerest ersetzt ist; und
(c) das Auswählen einer unter (b) hergestellten Antikörpermutante, die eine höhere Bindungsaffinität für den Antikörper aus der zweiten Säugetierspezies aufweist als der speziesabhängige Antikörper.

9. Verfahren nach Anspruch 8, worin die erste Säugetierspezies ein Mensch ist.

10. Verfahren nach Anspruch 8, worin die zweite Säugetierspezies ein nichtmenschliches Säugetier ist.

11. Verfahren nach Anspruch 10, worin das nichtmenschliche Säugetier ein Primat ist.

12. Verfahren nach Anspruch 11, worin der nichtmenschliche Primat aus der aus Rhesusaffen, Javaneraffen, Pavianen, Schimpansen und Makaken bestehenden Gruppe ausgewählt ist.

13. Verfahren nach Anspruch 8, worin der speziesabhängige Antikörper humanisierter MHM24-Anti-Human-CD11a ist und das Antigen menschliches CD11a ist.

14. Verfahren nach Anspruch 13, worin die zweite Säugetierspezies Rhesusaffen sind.

15. Verfahren nach Anspruch 8, worin Schritt (b) die Herstellung einer Mutante des speziesabhängigen Antikörpers umfasst, worin ein Rest, der unter (a) als an der Bindung des Homologs, nicht jedoch des Antigens aus der ersten Säugetierspezies beteiligt identifiziert wurde, durch einen anderen Aminosäurerest ersetzt wird.

16. Verfahren nach Anspruch 8, worin Schritt (b) die Herstellung einer Mutante des speziesabhängigen Antikörpers umfasst, worin ein Rest, der unter (a) als an der Bindung sowohl des Antigens aus der ersten Säugetierspezies als auch dessen Homologs beteiligt identifiziert wurde, durch einen anderen Aminosäurerest ersetzt wird.

17. Verfahren nach Anspruch 8, worin Schritt (b) die Herstellung einer Mutante des speziesabhängigen Antikörpers umfasst, worin ein Rest, der unter (a) als an der Bindung des Antigens aus der ersten Säugetierspezies, nicht jedoch dessen Homologs beteiligt identifiziert wurde, durch einen anderen Aminosäurerest ersetzt wird.

18. Variable Schwerkettendomäne, welche die Aminosäuresequenz der Seq.-ID Nr. 17 umfasst.

19. lsolierte Nucleinsäure, die für eine Antikörpermutante nach Anspruch 1 kodiert.

20. Vektor, der Nucleinsäure nach Anspruch 19 umfasst.

21. Wirtszelle, die mit einem Vektor nach Anspruch 20 transformiert ist.

22. Verfahren zur Herstellung einer Antikörpermutante, umfassend das Kultivieren einer Wirtszelle nach Anspruch 21 auf solche Weise, dass die Nucleinsäure exprimiert wird.

23. Verfahren nach Anspruch 22, das weiters das Gewinnen der Antikörpermutante aus der Wirtszellkultur umfasst.

24. Verfahren nach Anspruch 23, worin die Antikörpermutante aus dem Wirtszellkulturmedium gewonnen wird.

## Revendications

1. Mutant d'anticorps d'un anticorps MHM24 humanisé anti-CD11a humain, anticorps mutant qui comprend un domaine variable de chaîne lourde comprenant la séquence d'aminoacides figurant dans la SEQ ID N° 17 et qui possède une affinité de liaison pour CD11a provenant d'un mammifère non humain qui est au moins environ 10 fois supérieure à l'affinité de liaison de l'anticorps MHM24 humanisé pour ledit CD11a.

2. Mutant d'anticorps suivant la revendication 1, dans lequel le mammifère non humain est un primate.

3. Mutant d'anticorps suivant la revendication 2, dans lequel le primate non humain est choisi dans le groupe consistant en un singe rhésus, un singe cynomolgus, un babouin, un chimpanzé et un macaque.

4. Mutant d'anticorps suivant la revendication 1, le mutant d'anticorps ayant une affinité de liaison pour ledit CD11a provenant du mammifère non humain qui est au moins environ 20 fois supérieure à l'affinité de liaison de l'anticorps MHM24 humanisé anti-CD11a humain pour ledit CD11a.

5. Mutant d'anticorps suivant la revendication 1, le mutant d'anticorps ayant une affinité de liaison pour ledit CD11a provenant du mammifère non humain qui est au moins environ 50 fois supérieure à l'affinité de liaison de l'anticorps MHM24 humanisé anti-CD11a humain pour ledit CD11a.

6. Mutant d'anticorps suivant la revendication 1, comprenant en outre une substitution d'aminoacide dans une région d'ossature dudit anticorps MHM24 humanisé anti-CD11a humain.

7. Méthode non thérapeutique d'utilisation de l'anticorps mutant de la revendication 1, dans des études précliniques par administration de celui-ci à un mammifère non humain.

8. Procédé pour la production d'un mutant d'anticorps, comprenant les étapes consistant :
(a) à identifier des résidus de région hypervariable dans un anticorps dépendant d'une espèce qui sont impliqués dans la liaison d'un antigène provenant d'une première espèce de mammifère et les résidus de région hypervariable impliqués dans la liaison d'un homologue de l'antigène provenant d'une seconde espèce de mammifère, différente ;
(b) à préparer un mutant de l'anticorps dépendant de l'espèce, dans lequel un résidu identifié en (a) en tant que résidu impliqué dans la liaison de l'antigène provenant de la première espèce de mammifère ou de son homologue, ou bien des deux, est remplacé par un autre résidu d'aminoacide ; et
(c) à sélectionner un mutant d'anticorps préparé comme en (b) qui a une affinité de liaison pour l'antigène provenant de la seconde espèce de mammifère supérieure à celle de l'anticorps dépendant de l'espèce.

9. Procédé suivant la revendication 8, dans lequel la première espèce de mammifère est un être humain.

10. Procédé suivant la revendication 8, dans lequel la seconde espèce de mammifère est un mammifère non humain.

11. Procédé suivant la revendication 10, dans lequel le mammifère non humain est un primate.

12. Procédé suivant la revendication 11, dans lequel le primate non humain est choisi dans le groupe consistant en un singe rhésus, un singe cynomolgus, un babouin, un chimpanzé et un macaque.

13. Procédé suivant la revendication 8, dans lequel l'anticorps dépendant de l'espèce est l'anticorps MHM24 humanisé anti-CD11a humain et l'antigène est le CD11a humain.

14. Procédé suivant la revendication 13, dans lequel la seconde espèce de mammifère est un singe rhésus.

15. Procédé suivant la revendication 8, dans lequel l'étape (b) comprend la préparation d'un mutant de l'anticorps dépendant de l'espèce, un résidu identifié en (a) en tant que résidu impliqué dans les liaisons de l'homologue, mais non de l'antigène provenant de la première espèce de mammifère, étant remplacé par un autre résidu d'aminoacide.

16. Procédé suivant la revendication 8, dans lequel l'étape (b) comprend la préparation d'un mutant de l'anticorps dépendant de l'espèce, un résidu identifié en (a) en tant que résidu impliqué dans les liaisons à la fois de l'antigène provenant de la première espèce de mammifère et de son homologue, étant remplacé par un autre résidu d'aminoacide.

17. Procédé suivant la revendication 8, dans lequel l'étape (b) comprend la préparation d'un mutant de l'anticorps dépendant de l'espèce, un résidu identifié en (a) en tant que résidu impliqué dans les liaisons de l'antigène provenant de la première espèce de mammifère, mais non de son homologue, étant remplacé par un autre résidu d'aminoacide.

18. Domaine variable de chaîne lourde comprenant la séquence d'aminoacides figurant dans la SEQ ID N° 17.

19. Acide nucléique isolé codant pour le mutant d'anticorps de la revendication 1.

20. Vecteur comprenant l'acide nucléique de la revendication 19.

21. Cellule hôte transformée avec le vecteur de la revendication 20.

22. Procédé pour la production d'un mutant d'anticorps, comprenant la culture de la cellule hôte de la revendication 21, de telle sorte que l'acide nucléique soit exprimé.

23. Procédé suivant la revendication 22, comprenant en outre l'étape consistant à recueillir le mutant d'anticorps à partir de la culture de cellules hôtes.

24. Procédé suivant la revendication 23, dans lequel le mutant d'anticorps est recueilli à partir du milieu de culture des cellules hôtes.


### SEQUENCE LISTING

(1) GENERAL INFORMATION:
   (i) APPLICANT: Genentech, Inc.
   (ii) TITLE OF INVENTION: Antibody Mutants
   (iii) NUMBER OF SEQUENCES: 17
   (iv) CORRESPONDENCE ADDRESS:
      (A) ADDRESSEE: Genentech, Inc.
      (B) STREET: 1 DNA Way
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Lee, Wendy M.
      (B) REGISTRATION NUMBER: 40,378
      (C) REFERENCE/DOCKET NUMBER: P1064PCT
   (ix) TELECOMMUNICATION INFORMATION:
      (A) TELEPHONE: 650/225-1994
      (B) TELEFAX: 650/952-9881
(2) INFORMATION FOR SEQ ID NO:1:
   (i) SEQUENCE CHARACTERISTICS:
      (A) LENGTH: 108 amino acids
      (B) TYPE: Amino Acid
      (D) TOPOLOGY: Linear
   (xi) SEQUENCE DESCRIPTION: SEQ ID NO:1:
   Asp Val Gln Ile Thr Gln Ser Pro Ser Tyr Leu Ala Ala Ser Pro
     1               5                  10                  15
   Gly Glu Thr Ile Ser Ile Asn Cys Arg Ala Ser Lys Thr Ile Ser
                    20                  25                  30
   Lys Tyr Leu Ala Trp Tyr Gln Glu Lys Pro Gly Lys Thr Asn Lys
                    35                  40                  45
   Leu Leu Ile Tyr Ser Gly Ser Thr Leu Gln Ser Gly Ile Pro Ser
                    50                  55                  60
   Arg Phe Ser Gly Ser Gly Ser Gly Thr Asp Phe Thr Leu Thr Ile
                   65                  70                  75
   Ser Ser Leu Glu Pro Glu Asp Phe Ala Met Tyr Tyr Cys Gln Gln
                   80                  85                  90
   His Asn Glu Tyr Pro Leu Thr Phe Gly Thr Gly Thr Lys Leu Glu
                   95                  100                 105
   Leu Lys Arg
           108
(2) INFORMATION FOR SEQ ID NO:2:
   (i) SEQUENCE CHARACTERISTICS:
      (A) LENGTH: 108 amino acids
      (B) TYPE: Amino Acid
      (D) TOPOLOGY: Linear
   (xi) SEQUENCE DESCRIPTION: SEQ ID NO:2:
   Asp Ile Gln Met Thr Gln Ser Pro Ser Ser Leu Ser Ala Ser Val
    1              5                   10                  15
   Gly Asp Arg Val Thr Ile Thr Cys Arg Ala Ser Lys Thr Ile Ser
                   20                  25                  30
   Lys Tyr Leu Ala Trp Tyr Gln Gln Lys Pro Gly Lys Ala Pro Lys
                   35                  40                  45
   Leu Leu Ile Tyr Ser Gly Ser Thr Leu Gln Ser Gly Val Pro Ser
                   50                  55                  60
   Arg Phe Ser Gly Ser Gly Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   His Asn Glu Tyr Pro Leu Thr Phe Gly Gln Gly Thr Lys Val Glu
                   95                  100                 105
   Ile Lys Arg
           108
(2) INFORMATION FOR SEQ ID NO:3:
   (i) SEQUENCE CHARACTERISTICS:
      (A) LENGTH: 108 amino acids
      (B) TYPE: Amino Acid
      (D) TOPOLOGY: Linear
   (xi) SEQUENCE DESCRIPTION: SEQ ID NO:3:
   Asp Ile Gln Met Thr Gln Ser Pro Ser Ser Leu Ser Ala Ser Val
    1               5                  10                  15
   Gly Asp Arg Val Thr Ile Thr Cys Arg Ala Ser Gln Ser Ile Ser
                   20                  25                  30
   Asn Tyr Leu Ala Trp Tyr Gln Gln Lys Pro Gly Lys Ala Pro Lys
                   35                  40                  45
   Leu Leu Ile Tyr Ala Ala Ser Ser Leu Glu Ser Gly Val Pro Ser
                   50                  55                  60
   Arg Phe Ser Gly Ser Gly Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   Tyr Asn Ser Leu Pro Trp Thr Phe Gly Gln Gly Thr Lys Val Glu
                   95                 100                 105
   Ile Lys Arg
           108
(2) INFORMATION FOR SEQ ID NO:4:
   (i) SEQUENCE CHARACTERISTICS:
      (A) LENGTH: 121 amino acids
      (B) TYPE: Amino Acid
      (D) TOPOLOGY: Linear
   (xi) SEQUENCE DESCRIPTION: SEQ ID NO : 4 :
   Glu Val Gln Leu Gln Gln Pro Gly Ala Glu Leu Met Arg Pro Gly
    1               5                  10                  15
   Ala Ser Val Lys Leu Ser Cys Lys Ala Ser Gly Tyr Ser Phe Thr
                   20                  25                  30
   Gly His Trp Met Asn Trp Val Arg Gln Arg Pro Gly Gln Gly Leu
                   35                  40                  45
   Glu Trp Ile Gly Met Ile His Pro Ser Asp Ser Glu Thr Arg Leu
                   50                  55                  60
   Asn Gln Lys Phe Lys Asp Lys Ala Thr Leu Thr Val Asp Lys Ser
                   65                  70                  75
   Ser Ser Ser Ala Tyr Met Gln Leu Ser Ser Pro Thr Ser Glu Asp
                   80                  85                  90
   Ser Ala Val Tyr Tyr Cys Ala Arg Gly Ile Tyr Phe Tyr Gly Thr
                   95                  100                 105
   Thr Tyr Phe Asp Tyr Trp Gly Gln Gly Thr Thr Leu Thr Val Ser
                   110                 115                 120
   Ser
   121
(2) INFORMATION FOR SEQ ID NO:5:
   (i) SEQUENCE CHARACTERISTICS:
      (A) LENGTH: 121 amino acids
      (B) TYPE: Amino Acid
      (D) TOPOLOGY: Linear
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 113 amino acids
      (B) TYPE: Amino Acid
      (D) TOPOLOGY: Linear
   (xi) SEQUENCE DESCRIPTION: SEQ ID NO:6:
   Glu Val Gln Leu Val Glu Ser Gly Gly Gly Leu Val Gln Pro Gly
   1 5 10 15
   Gly Ser Leu Arg Leu Ser Cys Ala Ala Ser Gly Phe Thr Phe Ser
   20 25 30
   Ser Tyr Ala Met Ser Trp Val Arg Gln Ala Pro Gly Lys Gly Leu
   35 40 45
   Glu Trp Val Ser Val Ile Ser Gly Asp Gly Gly Ser Thr Tyr Tyr
   50 55 60
   Ala Asp Ser Val Lys Gly Arg Phe Thr Ile Ser Arg Asp Asn Ser
   65 70 75
   Lys Asn Thr Leu Tyr Leu Gln Met Asn Ser Leu Arg Ala Glu Asp
                   80                  85                  90
   Thr Ala Val Tyr Tyr Cys Ala Arg Gly Phe Asp Tyr Trp Gly Gln
                   95                  100                 105
   Gly Thr Leu Val Thr Val Ser Ser
                   110         113
(2) INFORMATION FOR SEQ ID NO:7:
   (i) SEQUENCE CHARACTERISTICS:
      (A) LENGTH: 184 amino acids
      (B) TYPE: Amino Acid
      (D) TOPOLOGY: Linear
   (xi) SEQUENCE DESCRIPTION: SEQ ID NO:7:
   Lys Gly Asn Val Asp Leu Val Phe Leu Phe Asp Gly Ser Met Ser
    1              5                   10                  15
   Leu Gln Pro Asp Glu Phe Gln Lys Ile Leu Asp Phe Met Lys Asp
                   20                  25                  30
   Val Met Lys Lys Leu Ser Asn Thr Ser Tyr Gln Phe Ala Ala Val
                   35                  40                  45
   Gln Phe Ser Thr Ser Tyr Lys Thr Glu Phe Asp Phe Ser Asp Tyr
                   50                  55                  60
   Val Lys Arg Lys Asp Pro Asp Ala Leu Leu Lys His Val Lys His
                   65                  70                  75
   Met Leu Leu Leu Thr Asn Thr Phe Gly Ala Ile Asn Tyr Val Ala
                   80                  85                  90
   Thr Glu Val Phe Arg Glu Glu Leu Gly Ala Arg Pro Asp Ala Thr
                   95                  100                 105
   Lys Val Leu Ile Ile Ile Thr Asp Gly Glu Ala Thr Asp Ser Gly
                   110                 115                 120
   Asn Ile Asp Ala Ala Lys Asp Ile Ile Arg Tyr Ile Ile Gly Ile
                   125                 130                 135
   Gly Lys His Phe Gln Thr Lys Glu Ser Gln Glu Thr Leu His Lys
                   140                 145                 150
   Phe Ala Ser Lys Pro Ala Ser Glu Phe Val Lys Ile Leu Asp Thr
                   155                 160                 165
   Phe Glu Lys Leu Lys Asp Leu Phe Thr Glu Leu Gln Lys Lys Ile
                   170                 175                 180
   Tyr Val Ile Glu
               184
(2) INFORMATION FOR SEQ ID NO:8:
   (i) SEQUENCE CHARACTERISTICS:
      (A) LENGTH: 184 amino acids
      (B) TYPE: Amino Acid
      (D) TOPOLOGY: Linear
   (xi) SEQUENCE DESCRIPTION: SEQ ID NO:8:
   Lys Gly Asn Val Asp Leu Ile Phe Leu Phe Asp Gly Ser Met Ser
    1               5                  10                  15
   Leu Gln Pro Asp Glu Phe Gln Lys Ile Leu Asp Phe Met Lys Asp
                   20                  25                  30
   Val Met Lys Lys Leu Ser Asn Thr Ser Tyr Gln Phe Ala Ala Val
                   35                  40                  45
   Gln Phe Ser Thr Ser Tyr Lys Thr Glu Phe Asp Phe Ser Asp Tyr
                   50                  55                  60
   Val Lys Gln Lys Asp Pro Asp Ala Leu Leu Glu His Val Lys His
                   65                  70                  75
   Met Leu Leu Leu Thr Asn Thr Phe Gly Ala Ile Asn Tyr Val Ala
                   80                  85                  90
   Thr Glu Val Phe Arg Glu Glu Leu Gly Ala Arg Pro Asp Ala Thr
                   95                  100                 105
   Lys Val Leu Ile Ile Ile Thr Asp Gly Glu Ala Thr Asp Ser Gly
                   110                 115                 120
   Asn Ile Asp Ala Ala Lys Asp Ile Ile Arg Tyr Ile Ile Gly Ile
                   125                 130                 135
   Gly Lys His Phe Gln Thr Lys Glu Ser Gln Glu Thr Leu His Lys
                   140                 145                 150
   Phe Ala Ser Lys Pro Ala Ser Glu Phe Val Lys Ile Leu Asp Thr
                   155                 160                 165
   Phe Glu Lys Leu Lys Asp Leu Phe Thr Glu Leu Gln Lys Lys Ile
                   170                 175                 180
   Tyr Ala Ile Glu
               184
(2) INFORMATION FOR SEQ ID NO:9:
   (i) SEQUENCE CHARACTERISTICS:
      (A) LENGTH: 7 amino acids
      (B) TYPE: Amino Acid
      (D) TOPOLOGY: Linear
   (xi) SEQUENCE DESCRIPTION: SEQ ID NO:9:
   Lys His Val Lys His Met Leu
   1 5 7
(2) INFORMATION FOR SEQ ID NO:10:
   (i) SEQUENCE CHARACTERISTICS:
      (A) LENGTH: 11 amino acids
      (B) TYPE: Amino Acid
      (D) TOPOLOGY: Linear
   (xi) SEQUENCE DESCRIPTION: SEQ ID NO:10:
   Pro Lys Asn Ser Ser Met Ile Ser Asn Thr Pro
   1 5 10 11
(2) INFORMATION FOR SEQ ID NO:11:
   (i) SEQUENCE CHARACTERISTICS:
      (A) LENGTH: 7 amino acids
      (B) TYPE: Amino Acid
      (D) TOPOLOGY: Linear
   (xi) SEQUENCE DESCRIPTION: SEQ ID NO:11:
   His Gln Ser Leu Gly Thr Gln
   1 5 7
(2) INFORMATION FOR SEQ ID NO:12:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:12:
   His Gln Asn Leu Ser Asp Gly Lys
   1 5 8
(2) INFORMATION FOR SEQ ID NO:13:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:13:
   His Gln Asn Ile Ser Asp Gly Lys
   1 5 8
(2) INFORMATION FOR SEQ ID NO:14 :
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:14:
   Val Ile Ser Ser His Leu Gly Gln
   1 5 8
(2) INFORMATION FOR SEQ ID NO:15:
   (i) SEQUENCE CHARACTERISTICS:
      (A) LENGTH: 26 base pairs
      (B) TYPE: Nucleic Acid
      (C) STRANDEDNESS: Single
      (D) TOPOLOGY: Linear
   (xi) SEQUENCE DESCRIPTION: SEQ ID NO:15:
      CACTTTGGAT ACCGCGTCCT GCAGGT 26
(2) INFORMATION FOR SEQ ID NO:16:
   (i) SEQUENCE CHARACTERISTICS:
      (A) LENGTH: 26 base pairs
      (B) TYPE: Nucleic Acid
      (C) STRANDEDNESS: Single
      (D) TOPOLOGY: Linear
   (xi) SEQUENCE DESCRIPTION: SEQ ID NO:16:
      CATCCTGCAG GTCTGCCTTC AGGTCA 26
(2) INFORMATION FOR SEQ ID NO:17:
   (i) SEQUENCE CHARACTERISTICS:
      (A) LENGTH: 121 amino acids
      (B) TYPE: Amino Acid
      (D) TOPOLOGY: Linear
   (xi) SEQUENCE DESCRIPTION: SEQ ID NO:17:
   Glu Val Gln Leu Val Glu Ser Gly Gly Gly Leu Val Gln Pro Gly
   1 5 10 15
   Gly Ser Leu Arg Leu Ser Cys Ala Ala Ser Gly Tyr Ser Phe Thr
   20 25 30
   Gly His Trp Met Asn Trp Val Arg Gln Ala Pro Gly Lys Gly Leu
   35 40 45
   Glu Trp Val Gly Met Ile Ala Pro Ala Ser Ser Ser Thr Arg Tyr
   50 55 60
   Asn Gln Lys Phe Lys Asp Arg Phe Thr Ile Ser Val Asp Lys Ser
   65 70 75
   Lys Asn Thr Leu Tyr Leu Gln Met Asn Ser Leu Arg Ala Glu Asp
   80 85 90
   Thr Ala Val Tyr Tyr Cys Ala Arg Gly Ile Tyr Phe Tyr Gly Thr
   95 100 105
   Thr Tyr Phe Asp Tyr Trp Gly Gln Gly Thr Leu Val Thr Val Ser
   110 115 120
   Ser
   121